# EUROPÄISCHE PATENTSCHRIFT

(11) **EP 3 160 932 B1**
(45) Veröffentlichungstag und Bekanntmachung des Hinweises auf die Patenterteilung: **14.03.2018**
(21) Anmeldenummer: 15729525.4
(22) Anmeldetag: 22.06.2015
(51) Int. Cl.: C07C 201/08, C07C 205/06, B01J 19/00

(54) **VERFAHREN ZUR HERSTELLUNG VON NITROBENZOL**
PROCESS FOR PRODUCING NITROBENZENE
PROCÉDÉ DE PRODUCTION DE NITROBENZÈNE

(30) Priorität: 24.06.2014 EP 14173583
(43) Veröffentlichungstag der Anmeldung: 03.05.2017
(73) Patentinhaber: Covestro Deutschland AG, 51373 Leverkusen (DE)
(72) Erfinder: KNAUF, Thomas, 41542 Dormagen (DE); DRINDA, Peter, 47829 Krefeld (DE)
(74) Vertreter: Levpat
(86) Internationale Anmeldenummer: PCT/EP2015/063924
(87) Internationale Veröffentlichungsnummer: WO 2015/197521

(56) Entgegenhaltungen:
- WO-A1-2013/113651

## Beschreibung

Die vorliegende Erfindung betrifft ein Verfahren zur kontinuierlichen Herstellung von Nitrobenzol durch Nitrierung von Benzol mit einem Gemisch aus Salpetersäure und Schwefelsäure, bei dem während eines Produktionsstillstandes nicht die ganze Produktionsanlage abgefahren wird, sondern die Produktionsanlage ganz oder zumindest teilweise "im Kreis" gefahren wird. Des Weiteren betrifft die vorliegende Erfindung eine Anlage zur Herstellung von Nitrobenzol und ein Verfahren zum Betrieb einer Anlage zur Herstellung von Nitrobenzol.

Nitrobenzol ist ein wichtiges Zwischenprodukt der chemischen Industrie, das insbesondere zur Herstellung von Anilin und damit auch zur Herstellung der Di- und Polyisocyanate der Diphenylmethanreihe und den darauf basierenden Polyurethanen benötigt wird.

Die Nitrierung von Benzol mit Salpetersäure zu einem Roh-Nitrobenzol war bereits Gegenstand zahlreicher Veröffentlichungen und Patentanmeldungen. Die heute gängigen Verfahren entsprechen im Wesentlichen dem Konzept der adiabaten Nitrierung von Benzol durch ein Gemisch von Schwefel- und Salpetersäure, welches im Allgemeinen als Mischsäure bezeichnet wird. Ein solches Verfahren wurde erstmals in US 2,256,999 beansprucht und ist in heutigen Ausführungsformen beispielsweise in EP 0 436 443 B1, EP 0 771 783 B1 und US 6 562 247 B2 beschrieben. Die Verfahren mit adiabater Reaktionsführung zeichnen sich insbesondere dadurch aus, dass keine technischen Maßnahmen ergriffen werden, um der Reaktionsmischung Wärme zu- oder abzuführen.

Auch sind isotherme Verfahren zur Nitrierung von Benzol mit Mischsäure bekannt, wie beispielsweise in EP 0 156 199 B1 beschrieben.

Auch sind Verfahren beispielsweise aus US 2 739 174 oder US 3 780 116 zur Nitrierung von Benzol bekannt, die ohne den Einsatz von Schwefelsäure auskommen.

Grundsätzlich sind auch Gasphasenverfahren zur Nitrierung von Benzol mit Salpetersäure oder Stickstoffoxiden möglich, jedoch sind die dadurch erzielbaren Ausbeuten zum gegenwärtigen Zeitpunkt noch gering (EP 0 078 247 B1, EP 0 552 130 B1).

WO 2013/113651 A1 beschreibt ein Verfahren und eine Anlage zur insbesondere kontinuierlichen Herstellung von Nitrobenzol mittels adiabater Nitrierung von Benzol mit Salpetersäure in Gegenwart von Schwefelsäure, in dem im Anschluss an die Nitrierung eine mehrstufige Aufkonzentrierung der Schwefelsäure mittels Erwärmen bei gegenüber Umgebungsdruck reduziertem Druck ausgeführt wird und wobei das Erwärmen durch Ausnutzung der in der adiabaten Nitrierung von Benzol erzeugten Wärme erfolgt.

All diesen Verfahren ist gemeinsam, dass als Reaktionsprodukt zunächst ein Roh-Nitrobenzol entsteht, das üblicherweise als Verunreinigungen Salpetersäure und - sofern mit Mischsäure nitriert wurde - Schwefelsäure, Wasser, Benzol, sowie als organische Verunreinigungen Dinitrobenzol, nitrierte Oxidationsprodukte des Benzols, insbesondere nitrierte Phenole (Nitrophenole) enthält. Das Roh-Nitrobenzol kann auch organische Verbindungen enthalten, die aus den Verbindungen entstanden, welche als Verunreinigungen im eingesetzten Benzol enthalten waren (WO 2008/148608 A1). Darüber hinaus enthält das Roh-Nihobenzol auch oft Metallsalze, die in den Säureresten oder im Roh-Nitrobenzol gelöst vorliegen können (DE 10 2007 059 513 A1). Diese Verunreinigungen sind unerwünscht, da sie Nachfolgeprozesse, in denen Nitrobenzol zum Einsatz kommt, wie zum Beispiel der Herstellung von Anilin, negativ beeinflussen können. Geeignete Aufarbeitungsverfahren, die Wasch- und Destillationsstufen beinhalten, sind beispielsweise in US 6,288,289 B1, EP 1 593 654 A1, EP 1 816 117 B1 und WO 2011/021057 A1 beschrieben.

Die Güte eines Reaktionsverfahrens zur Herstellung von Nitrobenzol ist also einerseits definiert durch den Gehalt des Roh-Produktes an unerwünschten Nebenkomponenten und Verunreinigungen, die durch unsachgemäße Reaktionsführung entstehen. Andererseits ist die Güte eines Reaktionsverfahrens dadurch definiert, dass der gesamte Prozess ohne technischen Produktionsausfall oder Probleme, die zu einem Eingriff in den Prozess nötigen, betrieben werden kann und dass Verluste an Einsatzstoffen vermieden oder zumindest minimal gehalten werden.

Solche Probleme können beispielsweise beim Anfahren bzw. Abfahren der Nitrierreaktion auftreten. Derartige Probleme können beispielsweise sein, dass es zur Entstehung von Feststoffen kommt, die zu Anbackungen und Verblockungen an der Ausrüstung (Nitrierkessel, Wärmetauschern, Leitungen, etc.) führen. Nachteilig ist des Weiteren, dass bei notwendigen Revisions-, Wartungs-, Reparatur- und Reinigungsarbeiten an oder in einem Reaktor oder einem sonstigen Anlagenteil regelmäßig immer alle Anlagenteile abgeschaltet werden müssen, da die Verfahrensschritte aufeinander aufbauen und somit immer sukzessive erfolgen. Dadurch muss die gesamte Anlage geleert werden, was zu einem erheblichen Materialausschuss führt. Des Weiteren muss Energie aufgewendet werden, um Reaktoren und Anlagenteile wieder auf die jeweiligen Betriebstemperaturen zu bringen. Solche Produktionsstillstände für Anlagenrevisionen, Reparatur- und Reinigungsmaßnahmen oder auftretende Rohstoff- oder Hilfsstoffmängel, geplant oder ungeplant, sind immer wiederkehrende Anlagenzustände, die einen erheblichen Einfluss auf das wirtschaftliche Betreiben einer kontinuierlich arbeitenden Anlage bzw. eines kontinuierlich arbeitenden Verfahrens haben.

Den beschriebenen Verfahren des Standes der Technik gelingt es zwar, mit hoher Ausbeute Nitrobenzol herzustellen, ohne dass es bei den Endprodukten zu einer Qualitätseinbuße kommt, jedoch werden nur Verfahren beschrieben, die sich im Normalbetrieb befinden. Produktionsstillstände für Anlagenrevisionen, Reparatur- und Reinigungsmaßnahmen oder z.B. Rohstoff- oder Hilfsstoffmangel werden nicht berücksichtigt. Dabei sind Produktionsstillstände, geplant oder ungeplant, immer wiederkehrende Anlagenzustände, die einen erheblichen Einfluss auf das wirtschaftliche Betreiben einer kontinuierlich arbeitenden Anlage haben.

Bei einem solchen Produktionsstillstand kann es sich um einen Revisionsstillstand halten, der im Voraus geplant wird, wobei zu diesem Zweck die Anlage abgefahren, die Energiezufuhren abgestellt und üblicherweise alle zu revidierenden Anlagenteile geöffnet und zum Zwecke der Prüfung gereinigt werden. Eine solche Revision kann eine oder mehrere Wochen dauern. Nach Beendigung der Revision wird die Produktionsanlage geschlossen, gegebenenfalls inertisiert, mit Hilfsstoffen versehen, und, nachdem die entsprechenden Energien und Rohstoffe zur Verfügung stehen, wieder angefahren. Ein Produktionsstillstand ist allerdings nicht zwangsläufig mit einem Öffnen oder einem anderen mechanischen Eingriff in einen Reaktor oder einen anderen Apparat der Anlage verbunden, sondern kann auch mit dem Abstellen und erneuten Starten der Produktionsanlage aus diversen anderen Gründen in Verbindung stehen, wie z. B. bei einem Ausfall der Rohstoffversorgung. In einem solchen Fall wird die Anlage üblicherweise im Teillastbetrieb gefahren und muss im schlimmsten Fall, wenn die logistische Versorgungskette unterbrochen ist, abgestellt werden. Des Weiteren können Produktionsstillstände durch Wartungs-, Reinigungs- oder Reparaturbedarf an der Produktionsanlage erzwungen sein. Hier spricht man üblicherweise von Kurzstillständen im Nitrobenzolprozess, wenn die Produktion bis zu einem Tag unterbrochen ist. Alle diese Produktionsstillstände zeichnen sich in der Praxis dadurch aus, dass es zu Produktionsverlusten kommt und dass beim Wiederanfahren der Anlage, wenn z. B. inertisiert werden muss, Stickstoff verbraucht wird oder beim Aufheizen der Anlage oder der Einsatzstoffe Energien wie Dampf und Strom benötigt werden.

Dem Fachmann ist bekannt, dass ein semikontinuierlich oder kontinuierlich betriebener industrieller Prozess ausgehend von einer in Betrieb befindlichen Produktionsanlage nicht augenblicklich in einen Produktionsstillstand überführt werden kann, sondern vorher kontrolliert abgefahren werden muss. Dies gilt auch für einen Anlagenausfall im Falle einer Havarie. Um nach dem Produktionsstillstand wieder produzieren zu können, muss die Anlage wieder auf die Prozessparameter vor dem Produktionsstillstand hochgefahren werden. Edukte und Apparaturen müssen aufgeheizt werden, Apparate müssen gegebenenfalls inertisiert werden, die Belastung der Apparate mit den Edukten wird allmählich auf den angestrebten Soll-Wert gesteigert. Während dieser Anfahrphase geht also weiterhin Produktionsmenge verloren, und es muss überproportional viel Energie aufgewendet werden, um die ausgekühlte Anlage zum Anfahren vorzubereiten und sie dann auch unter Beachtung aller betriebsrelevanten Parameter auf den gewünschten Sollwert hochzufahren.

Wünschenswert wäre also ein Verfahren zur Herstellung von Nitrobenzol, bei dem es durch einfache Maßnahmen ermöglicht wird, Produktionsstillstände beim Betreiben des Nitrobenzol-Herstellungsprozesses hinsichtlich Zeitaufwand, Energieverbrauch, Hilfsstoff- und Rohstoffverbrauch und/oder Verringerung von Abfällen zu optimieren. Dieses würde in nicht unerheblichen Ausmaß zu einer Verbesserung der Produktivität bzw. Wirtschaftlichkeit eines kontinuierlich betriebenen Verfahrens bzw. einer entsprechenden Produktionsanlage führen.

Überraschend wurde gefunden, dass diese Aufgabe für einen Nitrobenzol-Herstellprozess gelöst werden kann, wenn (vereinfacht ausgedrückt und ohne hierauf beschränkt zu sein) während eines Kurzstillstandes so viele Anlagenteile wie möglich in "Kreislauffahrweise" ("stand-by") gestellt werden, um nach der Maßnahme die Gesamtanlage unverzüglich wieder anfahren zu können. Überraschend wurde auch gefunden, dass der Energieverbrauch bei in für 1 Stunde bis zu 1 Tag in Kreislauf gestellter Anlage manchmal geringer ist als die Anlage komplett für einen Tag abzustellen und dann wieder anzufahren. Durch eine gezielte Kreislauffahrweise der nicht von dem Kurzstillstand betroffenen Anlagenteile werden vielfältige Vorteile realisiert, wie weiter unten noch näher erläutert wird.

Die vorliegende Erfindung stellt daher Folgendes bereit:
Ein, bevorzugt adiabatisch betriebenes, **Verfahren zur Herstellung von Nitrobenzol,** umfassend die Schritte
   (I) Nitrierung von Benzol mit Salpetersäure in Schwefelsäure unter Bildung von Nitrobenzol in einem Reaktor, wobei Benzol mit einem Massenstrom m₁, Salpetersäure mit einem Massenstrom m₂ und Schwefelsäure mit einem Massenstrom m₃ in den Reaktor eingetragen werden;
   (II) Phasentrennung des Reaktionsgemisches aus Schritt (I) in einem Phasentrennapparat in eine wässrige, Schwefelsäure enthaltende Phase und eine organische, Nitrobenzol enthaltende Phase; sowie optional (und bevorzugt) die Schritte (III) bis (VII):
      (III) Aufkonzentration der in Schritt (II) erhaltenen wässrigen Phase durch Verdampfung von Wasser in einem Verdampfungsapparat (dem sog. "Blitzverdampfer") zu einer wässrigen Schwefelsäure-haltigen Phase mit erhöhter Schwefelsäurekonzentration, und wobei die aufkonzentrierte, Schwefelsäure-haltige, wässrige Phase über einen Schwefelsäuretank in Schritt (I) zurückgeführt und als Bestandteil des Massenstroms m₃ eingesetzt wird;
      (IV) Mindestens zweistufiges Waschen der in Schritt (II) erhaltenen organischen, Nitrobenzol enthaltenden Phase und Abtrennung der wässrigen Phase nach jeder Stufe, wobei in jeder Stufe ein Waschbehälter mit Phasentrenneinrichtung oder ein Waschbehälter und ein separater Phasentrennapparat eingesetzt werden,
      (V) Destillation, bevorzugt Rektifikation der in der letzten Stufe von Schritt (IV) erhaltenen organischen, Nitrobenzol enthaltenden Phase in einem Destillationsapparat,
      (VI) Aufarbeitung des Abwassers der ersten Waschstufe von Schritt (IV) umfassend Sammeln des Abwassers in einem Abwassersammelbehälter und Reinigung dieses Abwassers in einer Vorrichtung zur Destillation oder Strippung,
      (VII) Aufarbeitung des Abwassers der zweiten Waschstufe von Schritt (IV) umfassend Sammeln des Abwassers in einem Abwassersammelbehälter und Reinigung dieses Abwassers in einer Vorrichtung zur Destillation oder Strippung, wobei der Vorrichtung zur Destillation oder Strippung eine Vorrichtung zur thermischen Druckzersetzung vor- und/oder nachgeschaltet sein kann, wobei bei einer Außerbetriebnahme eines oder mehrerer Anlagenteile aus den Schritten (I) bis (VII), insofern diese durchgeführt werden, der Massenstrom m₁ und der Massenstrom m₂ auf null reduziert werden und in mindestens einem der nicht außer Betrieb genommenen Anlagenteile der Ausgangsstrom wieder als Eingangsstrom des jeweiligen Anlagenteils oder eines davor liegenden Anlagenteils verwendet wird.

Ein weiterer Gegenstand der vorliegenden Erfindung ist eine **Anlage zur Herstellung von Nitrobenzol**, wie sie weiter unten noch im Detail beschrieben wird und die sich für die Durchführung des erfindungsgemäßen Verfahrens eignet.

Schließlich ist ein Gegenstand der vorliegenden Erfindung ein **Verfahren zum Betrieb einer Anlage zur Herstellung von Nitrobenzol,** was weiter unten noch im Detail beschrieben wird.

Die *"Außerbetriebnahme"* eines Anlagenteils meint dabei dessen Stilllegung, so dass in dem Anlagenteil eine Revisions-, Reparatur-, Wartungs- oder Reinigungsmaßnahme durchgeführt werden kann. Die vorliegende Erfindung ist also mit solchen Produktionsstillständen befasst, die man als "Kurzstillstände" zu Revisions-, Reparatur-, Reinigungs- oder Wartungszwecken in Teilen der Anlage oder z. B. infolge eines zeitlich begrenzten Mangels an Einsatzstoffen oder Hilfsstoffen bezeichnen kann. Die vorliegende Erfindung ermöglicht es, dass bei einer derartigen Maßnahme nicht die gesamte Produktionsanlage außer Betrieb genommen werden muss. Vielmehr ermöglicht es die vorliegende Erfindung, dass nicht von der Revisions-, Reparatur-, Wartungs- oder Reinigungsmaßnahme betroffene Anlagenteile bzw. die entsprechenden Verfahrensschritte in sog. "Kreislauffahrweise" betrieben werden können. Damit bleibt eine Komplettabschaltung der Anlage auf besondere (seltene) Fälle wie etwa den einer vollständigen Anlagenrevision beschränkt. Der Begriff *"Außerbetriebnahme"* umfasst demnach erfindungsgemäß bei Vorhandensein von *m Anlagenteilen im Sinne der vorliegenden Erfindung* (siehe hierzu auch den folgenden Absatz), wobei *m* eine natürliche Zahl ist, die Außerbetriebnahme von maximal *m* - 1 dieser Anlagenteile. Erfindungsgemäß wird also mindestens ein Anlagenteil **nicht** *"außer Betrieb genommen"* (d. h. *"vollständig stillgelegt"*). Bevorzugt befasst sich die vorliegende Erfindung mit dem Fall der Außerbetriebnahme von 1 bis 2 Anlagenteilen, besonders bevorzugt von 1 Anlagenteil. Erfindungsgemäß wird daher bei Außerbetriebnahme eines Anlagenteils (oder mehrerer Anlagenteile, jedoch nicht aller Anlagenteile) in jedem Fall die Bildung weiteren Produkts unterbrochen (da die Massenströme m₁ und m₂ auf null reduziert werden und daher kein weiteres Produkt mehr produziert werden kann). Erfindungsgemäß umfasst ist aber auch der Fall, dass der Reaktor aus Schritt (I) in *Kreislauffahrweise* (siehe hierzu auch den folgenden Absatz) betrieben und *ein anderes Anlagenteil* im Sinne der zuvor genannten Definition *außer Betrieb genommen* wird.

Unter *"Kreislauffahrweise"* wird im Rahmen dieser Erfindung verstanden, dass der Ausgangstrom eines Anlagenteils als Eingangsstrom dieses Anlagenteils oder eines anderen Anlagenteils, welcher dem betreffenden Anlagenteil unmittelbar oder mittelbar (also mit weiteren Anlagenteilen dazwischen) vorgeschaltet ist, verwendet wird. Dabei ist mit *"Anlagenteil"* das dem jeweiligen Schritt (I) bis (V), insofern diese durchgeführt werden, entsprechende Anlagenteil einer Anlage zur Herstellung von Nitrobenzol durch das erfindungsgemäße Verfahren gemeint.

So umfasst beispielsweise das Anlagenteil aus Schritt (I) *"einen Reaktor"*, wobei dieser Begriff auch solche Ausführungsformen umfasst, in denen mehrere Reaktoren (z. B. eine Kaskade mehrere in Serie geschalteter Reaktoren) eingesetzt werden (mit anderen Worten, das Wort *"ein"* ist diesem Zusammenhang und im Zusammenhang mit anderen Apparaten auch anderer Anlagenteile als unbestimmter Artikel und nicht als Zahlwort aufzufassen). Parallel oder in Reihe geschaltete Reaktoren sind im Stand der Technik auch in der Herstellung von Nitrobenzol bekannt und können in bestimmten Dimensionierungen und betrieblichen Eigenarten auch Vorteile mit sich bringen. Ein Anlagenteil kann also mehrere, auch unterschiedliche (z. B. Waschbehälter und Phasentrennapparat in Schritt (IV)), Apparate umfassen. Das Anlagenteil aus Schritt (II) umfasst einen Phasentrennapparat, das Anlagenteil aus Schritt (III) einen Verdampfungsapparat, das Anlagenteil aus Schritt (IV) umfasst neben dem Rohnitrobenzoltank für jede Waschstufe einen Waschbehälter (entweder mit integrierter Einrichtung zur Phasentrennung oder gefolgt von einem separatem Phasentrennapparat), das Anlagenteil aus Schritt (V) eine Destillationsapparatur. Es versteht sich von selbst, dass die Anlagenteile neben den zuvor explizit aufgeführten Apparaten auch Peripheriegeräte wie etwa Pumpen, Wärmeaustauscher und dergleichen mehr umfassen können.

Die Kreislauffahrweise kann auch über mehrere Apparate eines Anlagenteils eingestellt werden. Beispielsweise kann der Ausgangsstrom des letzten Apparats mehrerer in Serie geschalteter Apparate eines Anlagenteils als Eingangsstrom des ersten Apparats der in Serie geschalteten Apparate dieses Anlagenteils verwendet werden. Es ist auch möglich, die Kreislauffahrweise nur auf einen Teil der Apparate eines Anlagenteils anzuwenden, z. B. wenn der Ausgangsstrom des letzten Apparats mehrerer in Serie geschalteter Apparate eines Anlagenteils nicht in den ersten, sondern in einen weiteren Apparat dieses Anlagenteils zurückgeführt wird.

Die Kreislauffahrweise kann auch über mehrere Anlagenteile eingestellt werden. Beispielsweise kann der Ausgangsstrom des letzten Apparats eines Anlagenteils, z. B. der in der Destillation in Schritt (V) erhaltene Nitrobenzolstrom, als Eingangsstrom des ersten Apparats eines davor liegenden Anlagenteils, z. B. der Wäsche in Schritt (IV), verwendet werden, wobei die Kreislauffahrweise eingestellt wird, indem dann der Ausgangsstrom der beispielhaft genannten Destillation als Eingangsstrom der Wäsche dient.

Durch das Abstellen von m₁ und m₂, also der Massenströme von Benzol und Salpetersäure in den Reaktor aus Schritt (I), wird sichergestellt, dass während der Unterbrechung, die wie oben beschrieben zum Zwecke der Revision, Wartung, Reparatur und/oder Reinigung eines Teiles der Herstellungsanlage durchgeführt wird oder durch einen Mangel an Rohstoff(en) und/oder Hilfsstoffe(n) verursacht wurde, die Reaktion im Schritt (I) nicht weiter stattfindet. Dadurch und durch die Verwendung des Ausgangsstromes mindestens eines nicht unterbrochenen Schrittes und zugehörigen Anlagenteils als Eingangsstrom des jeweiligen Schrittes und zugehörigen Anlagenteils oder eines davorliegenden Anlagenteils wird sichergestellt, dass diese Schritte und zugehörige Anlagenteile jeweils "im Kreis" gefahren werden.

Ausführungsformen des erfindungsgemäßen Verfahrens werden nachfolgend beschrieben. Sie können beliebig miteinander kombiniert werden, sofern sich aus dem Kontext nicht eindeutig das Gegenteil ergibt.

Die einzelnen Schritte des erfindungsgemäßen Verfahrens werden vorzugsweise im Rahmen eines kontinuierlichen Prozesses durchgeführt.

Es ist besonders bevorzugt, dass das erfindungsgemäße Verfahren auch die Schritte (III), (IV), (V), (VI) und (VII) umfasst.

Der gesamte Prozess der Nitrobenzol-Herstellung kann gedanklich wie folgt aufgeteilt werden:
1.) Nitrierreaktion (Schritt (I) und zugehöriges Anlagenteil) mit Phasentrennung (Schritt (II) und zugehöriges Anlagenteil) und Aufkonzentrierung der Schwefelsäure (Schritt (III) und zugehöriges Anlagenteil),
2.) Wäschen (bevorzugt saure, alkalische und neutrale Wäsche; Schritt (IV) und zugehöriges Anlagenteil),
3.) Destillation (Schritt (V) und zugehöriges Anlagenteil) sowie
4.) optional (und bevorzugt) saure Abwasseraufarbeitung (Schritt (VI) und zugehöriges Anlagenteil) und
5.) optional (und bevorzugt) alkalische Abwasseraufarbeitung (Schritt (VII) und zugehöriges Anlagenteil).

Bevorzugt umfasst dabei der Schritt (IV) die Schritte
(IVa) Waschen der in Schritt (II) erhaltenen organischen, Nitrobenzol enthaltenen Phase in mindestens einer sauren Wäsche und anschließende Phasentrennung in eine wässrige und eine organische, Nitrobenzol enthaltenden Phase (1. Waschstufe);
(IVb) Waschen der in Schritt (IVa) erhaltenen organischen Phase in mindestens einer alkalischen Wäsche mit einer wässrigen Lösung einer Base, die ausgewählt ist aus der Gruppe bestehend aus Natriumhydroxid, Natriumcarbonat und Natriumhydrogencarbonat und anschließende Phasentrennung in eine wässrige und eine organische, Nitrobenzol enthaltende Phase (2. Waschstufe);
(IVc) Waschen der in Schritt (IVb) erhaltenen organischen Phase in mindestens einer, bevorzugt zwei bis vier, besonders bevorzugt zwei bis drei, ganz besonders bevorzugt zwei neutralen Wäschen mit Wasser und anschließender Phasentrennung in eine wässrige und eine organische, Nitrobenzol enthaltende organische Phase (3. Waschstufe).

In einer bevorzugten Ausführungsform umfasst das erfindungsgemäße Verfahren daher die Schritte
(I) Nitrierung von Benzol mit Gemischen aus Salpetersäure und Schwefelsäure unter Bildung von Nitrobenzol in einem Reaktor, wobei Benzol mit einem Massenstrom m₁ und ein Gemisch aus Salpetersäure (Massenstrom m₂) und Schwefelsäure (Massenstrom m₃) in den Reaktor eingetragen werden;
(II) Phasentrennung des Reaktionsgemisches aus Schritt (I) in einem Phasentrennapparat in eine wässrige und eine organische, Nitrobenzol enthaltende Phase,
(III) Aufkonzentration der Schwefelsäure enthaltenden wässrigen Phase aus Schritt (II) in einem Verdampfungsapparat zu einer wässrigen Schwefelsäure-haltigen Phase mit erhöhter Schwefelsäurekonzentration, und wobei die aufkonzentrierte, Schwefelsäure-haltige, wässrige Phase über einen Schwefelsäuretank in Schritt (I) zurückgeführt und als Bestandteil des Massenstroms m₃ eingesetzt wird;
(IV) Mindestens zweistufiges Waschen der in Schritt (II) erhaltenen organischen, Nitrobenzol enthaltenden Phase, wobei in jeder Stufe ein Waschbehälter mit Phasentrenneinrichtung oder ein Waschbehälter und ein separater Phasentrennapparat eingesetzt werden, umfassend
(IVa) Waschen der in Schritt (II) erhaltenen organischen, Nitrobenzol enthaltenden Phase in mindestens einer, bevorzugt einer bis zwei, besonders bevorzugt einer Wäsche(n) (sog. "saure Wäsche(n)") und anschließende Phasentrennung in eine wässrige und eine organische, Nitrobenzol enthaltende Phase,
(IVb) Waschen der in Schritt (IVa) erhaltenen organischen, Nitrobenzol enthaltenden Phase in mindestens einer, bevorzugt einer bis zwei, besonders bevorzugt einer alkalischen Wäsche(n) mit einer wässrigen Lösung einer Base ausgewählt aus der Gruppe bestehend aus Natriumhydroxid, Natriumcarbonat und Natriumhydrogencarbonat,
   und anschließende Phasentrennung in eine wässrige und eine organische, Nitrobenzol enthaltende Phase,
(IVc) Waschen der in Schritt (IVb) erhaltenen organischen, Nitrobenzol enthaltenden Phase in mindestens einer, bevorzugt zwei bis vier, besonders bevorzugt zwei bis drei, ganz besonders bevorzugt zwei neutralen Wäsche(n) mit Wasser und anschließende Phasentrennung in eine wässrige und eine organische, Nitrobenzol enthaltende Phase,
(V) Destillation, bevorzugt Rektifikation der in Schritt (IVc) erhaltenen, organischen, Nitrobenzol enthaltenden Phase, wobei gereinigtes Nitrobenzol erhalten wird,
(VI) Aufarbeitung des Abwassers Schritt (IVa) umfassend Sammeln des Abwassers in einem Abwassersammelbehälter und Reinigung dieses Abwassers in einer Vorrichtung zur Destillation oder Strippung, und
(VII) Aufarbeitung des Abwassers aus Schritt (IVb) umfassend Sammeln des Abwassers in einem Abwassersammelbehälter und Reinigung dieses Abwassers in einer Vorrichtung zur Destillation oder Strippung, wobei der Vorrichtung zur Destillation oder Strippung eine Vorrichtung zur thermischen Druckzersetzung vor- und/oder nachgeschaltet sein kann,
   wobei bei einer Außerbetriebnahme eines oder mehrerer Anlagenteile aus den Schritten (I) bis (VII) der Massenstrom m₁ und der Massenstrom m₂ auf null reduziert werden und in mindestens einem der nicht außer Betrieb genommenen Anlagenteile der Ausgangsstrom wieder als Eingangsstrom des jeweiligen Anlagenteils oder eines davor liegenden Anlagenteils verwendet wird.

Unter *thermischer Druckzersetzung* wird dabei ein Verfahren zur Aufarbeitung alkalischen Abwassers verstanden, bei dem organische Verunreinigungen unter Einwirkung von erhöhtem Druck und erhöhter Temperatur zersetzt werden. Geeignete Verfahren sind dem Fachmann bekannt und beispielsweise in EP 1 593 654 B1 beschrieben. Im Rahmen der vorliegenden Erfindung ist es insbesondere bevorzugt, das (ggf. in der Vorrichtung zur Destillation oder Strippung vorbehandelte) alkalische Abwasser unter Ausschluss von Sauerstoff auf Temperaturen von 150 °C bis 500 °C unter einem absoluten Druck von 50 bar bis 350 bar zu erhitzen.

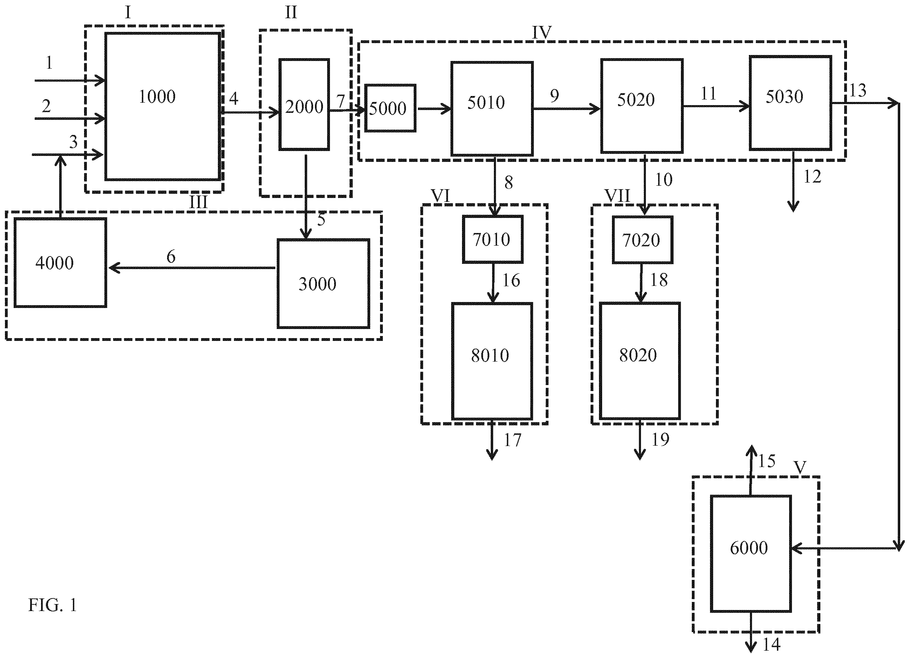

Die beigefügte Zeichnung FIG. 1 zeigt eine Ausführungsform mit drei Waschstufen (ohne die optionale thermische Druckzersetzung) mit Prozessströmen im Regelbetrieb:
Benzol (1), Salpetersäure (2) und Schwefelsäure (3) werden in einem Reaktor (1000) umgesetzt. Das Verfahrensprodukt dieses Schrittes (I), Strom 4, wird in einem Phasentrennapparat (2000) in eine wässrige (5) und eine organische, Nitrobenzol enthaltende Phase (7) aufgetrennt (Schritt (II)). Die wässrige Phase (5), die im Wesentlichen aus verdünnter Schwefelsäure besteht, wird in einem Verdampfungsapparat (3000) aufkonzentriert (Schritt (III)). Die so erhaltene, aufkonzentrierte, Schwefelsäure-haltige Phase wird über einen Schwefelsäuretank (4000) in den Reaktor (1000) aus Schritt (I) zurückgeführt und als Bestandteil des Massenstroms m₃ eingesetzt. Die in Schritt (II) erhaltene organische Phase (7) wird über einen Rohnitrobenzoltank (5000) den einzelnen Waschstufen (5010 - "saure Wäsche"; 5020 - "alkalische Wäsche"; 5030 - "neutrale Wäsche") zugeführt, wobei die zugeführten Waschflüssigkeitsströme der Einfachheit halber nicht eingezeichnet wurden. Dabei werden die Abwasserströme 8, 10 und 12 sowie die gewaschene organische, Nitrobenzol enthaltende Phase 13 erhalten (Schritt (IV) mit den Teilschritten (IVa), (IVb) und (IVc)). Die organische Phase 13 wird in einer Destillationsapparatur (6000) gereinigt (Schritt (V)), wobei Leichtsieder (im Wesentlichen nicht umgesetztes Benzol) über Kopf (Strom 15) und Nitrobenzol am Sumpf (Strom 14) abgezogen werden. Dieser Leichtsiederabtrennung kann sich eine weitere destillative Reinigungsstufe (nicht gezeigt) anschließen, in welcher der Strom 14 zur Abtrennung von hochsiedenden Verunreinigungen über Kopf oder als Seitenstrom abgezogen wird.

Das Abwasser 8 der ersten Waschstufe (IVa) wird über einen Abwassersammelbehälter (7010) einer Reinigungsapparatur (8010) zugeführt (Strom 16), wobei gereinigtes Abwasser (17) erhalten wird (Schritt (VI)). Die Vorrichtung 8010 ist dabei eine Vorrichtung zur Destillation oder Strippung. Das Abwasser 10 der zweiten Waschstufe (IVb) wird über einen Abwassersammelbehälter (7020) einer Reinigungsapparatur (8020) zugeführt (Strom 18), wobei gereinigtes Abwasser (19) erhalten wird (Schritt (VII)). Die Vorrichtung 8020 ist dabei eine Vorrichtung zur Destillation oder Strippung. Wie bereits erwähnt, kann der Vorrichtung 8020 eine Vorrichtung zur thermischen Druckzersetzung vor- und/oder nachgeschaltet sein (nicht gezeigt in FIG. 1). Das Abwasser 12 der dritten Waschstufe (IVc) kann, wie aus dem Stand der Technik bekannt, entsorgt oder weiterverwendet werden (nicht gezeigt in FIG. 1).

Die beschriebene Ausführungsform betrifft insbesondere den Fall eines Kurzstillstandes der Produktion von 1 Stunde bis zu 1 Tag. Durch die erfindungsgemäße Vorgehensweise wird die Produktionsanlage komplett oder teilweise in Kreislauffahrweise einzelner Anlagenteile betrieben. Besonders bevorzugt ist es dabei, die Kreislauffahrweise jeweils über die oben definierten Bestandteile des Prozesses der Nitrobenzol-Herstellung zu führen. Dies bedeutet, dass (1) die Nitrierreaktion mit Phasentrennung und Aufkonzentrierung der Schwefelsäure, (2) die Wäschen (saure, alkalische und neutrale Wäsche), (3) die Destillation und (4) saure Abwasseraufarbeitung sowie (5) die alkalische Abwasseraufarbeitung jeweils in Kreislauffahrweise gestellt werden.

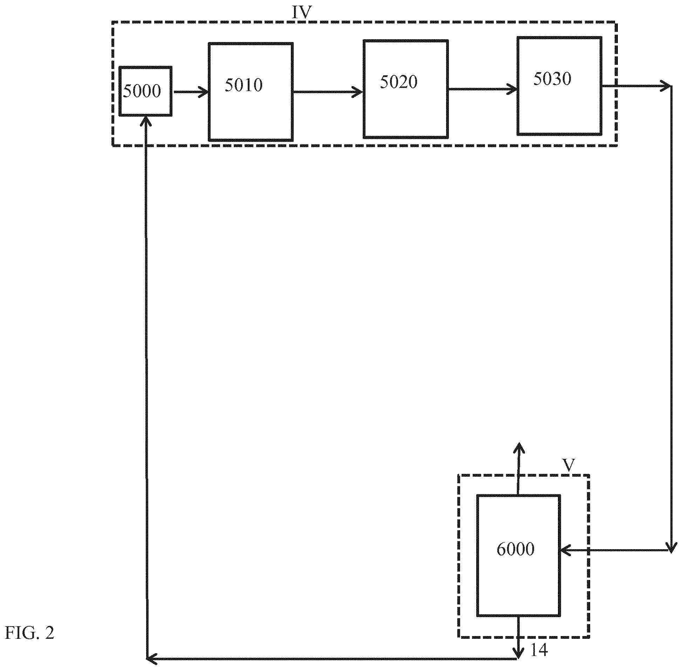

Bevorzugt ist dabei, wenn mindestens in den Schritten (IV) und (V) die Ausgangsströme wieder als Eingangsströme der dazugehörigen Anlagenteile verwendet werden. Dieses kann beinhalten, dass der Ausgangsstrom des Schrittes (IV) auch wieder als Eingangsstrom dieses Schrittes verwendet wird und bei Schritt (V) genauso verfahren wird. Alternativ können aber auch die Schritte (IV) und (V) zusammen in Kreislauffahrweise betrieben werden, d.h. der Ausgangsstrom von Schritt (IV) bildet den Eingangsstrom von Schritt (V), und der Ausgangsstrom von Schritt (V) bildet den Eingangsstrom von Schritt (IV). Diese Verfahrensweise ist schematisch und stark vereinfacht in FIG. 2 dargestellt. So kann beispielsweise die Destillation gemeinsam mit der Wäsche in Kreislauf gestellt werden, indem der Ausgang der Destillation (Nitrobenzol) in den Rohnitrotank und von dort über die Wäschen zurück zur Destillation geht.

Hierbei ist es insbesondere bevorzugt, wenn bei einer Unterbrechung des Verfahrens in mindestens einem der Schritte (I) bis (VII) (also der Außerbetriebnahme eines oder mehrerer Anlagenteile aus den Schritte (I) bis (VII)), wobei Schritt (IV) die oben genannten Teilschritte umfasst, in jedem anderen, d. h. nicht von der Unterbrechung betroffenen Schritt (I) bis (VII) der Ausgangsstrom wieder als Eingangsstrom des jeweiligen Anlagenteils verwendet wird. Dadurch kommen die erfindungsgemäßen Effekte besonders vorteilhaft zum Tragen.

Ein weiterer Gegenstand der vorliegenden Erfindung ist eine Anlage zur Herstellung von Nitrobenzol, umfassend die Anlagenteile:
(I) einen Reaktor zur Nitrierung von Benzol mit einem Gemisch aus Salpetersäure und Schwefelsäure unter Bildung von Nitrobenzol,
(II) einen Phasentrennapparat zur Phasentrennung des im Reaktor (I) erhaltenen Reaktionsgemisches in eine wässrige, Schwefelsäure enthaltende Phase und eine organische, Nitrobenzol enthaltende Phase, sowie optional (und bevorzugt) die Anlagenteile (III) bis (VII):
   (III) einen Verdampfer (III.a) zur Aufkonzentration der wässrigen, Schwefelsäure enthaltenden Phase und einen Schwefelsäurevorlagetank (III.b) zur Aufnahme der aufkonzentrierten, wässrigen, Schwefelsäure enthaltenden Phase und deren Bereitstellung für den Reaktor (I),
   (IV) einen Waschbehälter mit Phasentrenneinrichtung pro Waschstufe oder einen Waschbehälter und einen separaten Phasentrennapparat pro Waschstufe zum mindestens zweistufigen Waschen der organischen, Nitrobenzol enthaltenden Phase aus (II),
   (V) einen Destillationsapparat zur Reinigung der organischen, Nitrobenzol enthaltenden Phase aus (IV),
   (VI) einen Abwassersammelbehälter und eine Vorrichtung zur Destillation oder Strippung zum Sammeln und anschließenden Reinigen des Abwassers der ersten Waschstufe aus (IV),
   (VII) einen Abwassersammelbehälter und eine Vorrichtung zur Destillation oder Strippung, welcher eine Vorrichtung zur thermischen Druckzersetzung vor- und/oder nachgeschaltet sein kann, zum Sammeln und anschließenden Reinigen des Abwassers der zweiten Waschstufe aus (IV),
      wobei
      die Anlage derart ausgestaltet ist, dass bei einer Außerbetriebnahme eines oder mehrerer Anlagenteile (I) bis (VII), insofern diese vorhanden sind, kein weiterer Eintrag von Benzol und Salpetersäure in den Reaktor (I) stattfindet und unabhängig voneinander oder gleichzeitig in mindestens einem nicht von der Außerbetriebnahme betroffenen Anlagenteil der Ausgangsstrom zurückgeführt und als Eingangsstrom des jeweiligen Anlagenteils oder eines davor liegenden Anlagenteils verwendet wird. Die Ausgestaltung der Anlage derart, dass *"bei einer Außerbetriebnahme eines oder mehrerer Anlagenteile (I) bis* (*VII*), *insofern diese vorhanden sind, kein weiterer Eintrag von Benzol und Salpetersäure in den Reaktor (I) stattfindet"* ist dabei so zu verstehen, dass vor oder zeitgleich mit der Außerbetriebnahme eines Anlagenteils die Zufuhr von Benzol und Salpetersäure unterbrochen wird, d. h., dass vor oder zeitgleich mit dem Einstellen der Kreislauffahrweise in mindestens einem nicht von der Außerbetriebnahme betroffenen Anlagenteil, die Zufuhr von Benzol und Salpetersäure unterbrochen wird. **Vorrichtungstechnisch** lässt sich dies auf verschiedene Weisen realisieren, bspw. durch den Einbau prozessleittechnischer Einrichtungen, die die die Zufuhr von Benzol und Salpetersäure bei Außerbetriebnahme eines oder mehrerer Anlagenteile (bei Einstellen eines oder mehrerer der nicht außer Betrieb zu nehmenden Anlagenteile auf Kreislauffahrweise) automatisch unterbrechen. Die Einrichtung einer Sperrschaltung, die das Einstellen auf Kreislauffahrweise nur bei unterbrochener Zufuhr von Benzol und Salpetersäure ermöglicht, ist ebenfalls denkbar. Geeignete Soft- und Hardware-Produkte sind kommerziell erhältlich und dem Fachmann bekannt. Ggf. nötige Programmierungs- und Anpassungsarbeiten bewegen sich im Rahmen einer für den Fachmann üblichen Routinetätigkeit.

Dabei versteht sich, dass diese Anlage insbesondere dazu ausgestaltet ist, darin das erfindungsgemäße Verfahren durchführen zu können. Damit treffen die Vorteile und Effekte des erfindungsgemäßen Verfahrens auch auf die erfindungsgemäße Anlage zu.

Besonders bevorzugt umfasst das Anlagenteil (IV):
(IVa) einen Waschbehälter mit Phasentrenneinrichtung oder einen Waschbehälter und einen separaten Phasentrennapparat zur Wäsche und anschließenden Phasentrennung der organischen, Nitrobenzol enthaltenden Phase aus (II),
(IVb) einen Waschbehälter mit Phasentrenneinrichtung oder einen Waschbehälter und einen separaten Phasentrennapparat zur Wäsche und anschließenden Phasentrennung der organischen, Nitrobenzol enthaltenden Phase aus (IVa),
(IVc) einen Waschbehälter mit Phasentrenneinrichtung oder einen Waschbehälter und einen separaten Phasentrennapparat zur Wäsche und anschließenden Phasentrennung der organischen, Nitrobenzol enthaltenden Phase aus (IVb).

Dabei ist es insbesondere bevorzugt, dass unabhängig voneinander oder gleichzeitig in jedem anderen nicht von der Unterbrechung betroffenen Anlagenteil der Massenausgangsstrom zurückgeführt und wieder als Masseneingangsstrom des jeweiligen Anlagenteils oder eines davor liegenden Anlagenteils verwendet wird. Somit wird in den nicht von der Unterbrechung betroffenen Anlagenteilen mindestens eine Kreislauffahrweise durchgeführt.

Sollten dabei zwei oder mehr Nitrierreaktorlinien parallel betrieben werden, dann kann zunächst eine Reaktorlinie in erfindungsgemäßer Kreislauffahrweise betrieben werden und die anderen Reaktorlinien nacheinander auf die erfindungsgemäße Kreislauffahrweise eingestellt werden. Alternativ ist es im Rahmen der vorliegenden Erfindung aber auch möglich, alle NitrierReaktorlinien gleichzeitig oder im Wesentlichen gleichzeitig in die erfindungsgemäße Kreislauffahrweise zu überführen. Ebenfalls von den Verfahren und der Anlage gemäß der vorliegenden Erfindung sind Fälle umfasst, in denen 3 oder 4 Reaktorlinien in 1 oder 2 Phasentrennapparate gehen, 1 oder 2 Phasentrennapparate in 2 Blitzverdampfer gehen und gegebenenfalls die dort abfließende Schwefelsäure in einer Schwefelsäurevorlage gefahren wird. Die aus dem Phasentrennapparat auslaufende organische Phase kann beispielsweise in einer einstrassigen Wäsche mit Destillation aufgearbeitet werden.

Beispiele für verschiedene Kreislauffahrweisen sind im Folgenden aufgeführt:
1.) In der **Nitrierreaktion mit Phasentrennung und Aufkonzentrierung der Schwefelsäure,** bestehend aus Nitratoren mit Schwefelsäurekreislaufpumpen, Phasentrenner, Blitzverdampfer mit Vakuumpumpen und Kondensatoren und Schwefelsäurevorlage, werden zeitgleich der Benzolstrom und der Salpetersäurestrom abgestellt. Die Kreislaufschwefelsäure kann dann unter Reaktionsstartbedingungen (bevorzugt 101 °C) mittels der Schwefelsäurekreislaufpumpen durch die Nitratoren, Phasentrennung, Blitzverdampfer (Schwefelsäureaufkonzentrierung) in die Schwefelsäurevorlage und von dort im Kreis zurück zu den Nitratoren für mehrere Stunden gepumpt werden.
2.) In den **Wäschen**, bestehend aus einem Rohnitrobenzoltank, einer sauren, alkalischen und neutralen Wäsche, wird der Rohnitrobenzol-Strom aus dem Phasentrennapparat zum Rohnitrotank unterbrochen. Das gewaschene Rohnitrobenzol kann dann aus der neutralen Wäsche zum Rohnitrotank, über die saure Wäsche und alkalische Wäsche und zurück zur neutralen Wäsche für unbestimmte Zeit im Kreis gepumpt werden. Das Waschwasser wird im Gegenstrom in die neutrale über die alkalische Wäsche zur alkalischen Abwasseraufarbeitung gefahren. Dabei wird der Mengenstrom des Rohnitrobenzols aus dem Rohnitrobenzoltank zur sauren Wäsche und des Waschwassers zur neutralen Wäsche auf Minimallast reduziert.
3.) In der **Destillation,** bestehend aus einem Wärmetauscher, Nitrobenzol-Kolonne mit Sumpfentnahme des Endproduktes Nitrobenzol und einem Naturumlaufverdampfer, Brüdenkondensatoren mit Vakuumpumpen, Brüdenphasentrennapparat, Rückbenzolvorlage mit Pumpe, fällt nach der Abstellung der Reaktion kein Roh-Nitrobenzol in den Wäschen mehr an. Der Sumpfablauf der Nitrobenzol-Kolonne wird über den Wärmetauscher zurück auf den Rohnitrobenzoltank gestellt und somit über die gesamten Wäschen und über die Nitrobenzol-Kolonne im Kreis gefahren. Die anfallenden Brüden (Benzol und Wasser) werden kondensiert und im Brüdenphasentrennapparat in eine wässrige und organische Phase getrennt. Die Benzol enthaltende organische Phase wird ebenfalls zum Rohnitrobenzoltank geführt. Die wässrige Phase wird über die saure Wäsche und die saure Abwasseraufarbeitung entsorgt (das Vakuumsystem bleibt in Betrieb). Die Kreislauffahrweise kann unbestimmte Zeit betrieben werden.
4.) In der **sauren Abwasseraufarbeitung,** bestehend aus einem Abwasservorlagetank, einem Wärmeaustauscher, einer Abwasserdestillation mit Kondensationssystem, einem Abwasserkühler und Ablauf zur sauren Wäsche, fällt nach der Abstellung der Nitrierung kein saures Abwasser mehr an. Die Abwasserdestillation kann mit Dampf für unbestimmte Zeit betrieben werden, wobei das saure Abwasser im Sumpf der Abwasserkolonne zum Abwasservorlagetank in Kreislauffahrweise gestellt wird und die anfallenden Brüden zur sauren Wäsche gefahren werden.
5.) In der **alkalischen Abwasseraufarbeitung,** bestehend aus einem Abwasservorlagetank, einem Wärmetauscher, einer Abwasserdestillation mit Kondensationssystem, und Ablauf zu einer Anlage zur thermischen Druckzersetzung (TDZ), fällt nach der Abstellung der alkalischen Wäsche kein alkalisches Abwasser mehr an. Die Abwasserdestillation kann mit Dampf für unbestimmte Zeit betrieben werden, wobei das alkalische Abwasser im Sumpf der Abwasserkolonne zum Abwasservorlagetank in Kreislauffahrweise gestellt wird und die anfallenden Brüden zur sauren Wäsche gefahren werden. Die TDZ, bestehend aus Abwasservorlage mit Hochdruckpumpe, Wärmetauscher, Dampfheizer, Verweilzeitrohr und Kühler, kann mit Dampf für unbestimmte Zeit betrieben werden, wobei das alkalische Abwasser des Ausganges der TDZ in Kreislauffahrweise zur Abwasservorlage gestellt wird.

Diese Beispiele stehen natürlich nur exemplarisch für eine Vielzahl möglicher Kreislauffahrweisen, deren genaue Ausgestaltung von den konkreten Gegebenheiten einer Produktionsanlage abhängt und daher nicht verallgemeinert werden kann. Ein gemeinsames Merkmal aller denkbaren Kreislauffahrweisen ist jedoch, dass kein Produkt die Anlage verlässt, wenn es sich um eine einstrassige Nitrobenzol-Linie handelt.

Sollten zwei oder mehr Nitrobenzol-Reaktorlinien parallel betrieben werden, dann kann, muss aber nicht, Produkt die Anlage verlassen, wenn beispielsweise die Anlage mit Teillast gefahren wird.

Ein weiterer Gegenstand der vorliegenden Erfindung ist ein **Verfahren zum Betrieb einer Anlage zur Herstellung von Nitrobenzol,** umfassend die Anlagenteile:
(I) einen Reaktor zur Nitrierung von Benzol mit einem Gemisch aus Salpetersäure und Schwefelsäure unter Bildung von Nitrobenzol,
(II) einen Phasentrennapparat zur Phasentrennung des im Reaktor (I) erhaltenen Reaktionsgemisches in eine wässrige, Schwefelsäure enthaltende Phase und eine organische, Nitrobenzol enthaltende Phase, sowie optional (und bevorzugt) die Anlagenteile (III) bis (VII):
(III) einen Verdampfer (III.a) zur Aufkonzentration der wässrigen, Schwefelsäure enthaltenden Phase und einen Schwefelsäurevorlagetank (III.b) zur Aufnahme der aufkonzentrierten, wässrigen, Schwefelsäure enthaltenden Phase und deren Bereitstellung für den Reaktor (I),
(IV) einen Waschbehälter mit Phasentrenneinrichtung pro Waschstufe oder einen Waschbehälter und einen separaten Phasentrennapparat pro Waschstufe zum mindestens zweistufigen Waschen der organischen, Nitrobenzol enthaltenden Phase aus (II), wobei das Anlagenteil (IV) besonders bevorzugt die oben definierten Bestandteile (IVa), (IVb), und (IVc) umfasst,
(V) einen Destillationsapparat zur Reinigung der organischen, Nitrobenzol enthaltenden Phase aus (IV),
(VI) einen Abwassersammelbehälter und eine Vorrichtung zur Destillation oder Strippung zum Sammeln und anschließenden Reinigen des Abwassers der ersten Waschstufe aus (IV),
(VII) einen Abwassersammelbehälter und eine Vorrichtung zur Destillation oder Strippung, welcher eine Vorrichtung zur thermischen Druckzersetzung vor- und/oder nachgeschaltet sein kann, zum Sammeln und anschließenden Reinigen des Abwassers der zweiten Waschstufe aus (IV),
   wobei zur Außerbetriebnahme eines oder mehrerer Anlagenteile (I) bis (VII), insofern diese vorhanden sind, die folgenden Schritte durchlaufen werden:
   (i) Stoppen der Zufuhr von Benzol und Salpetersäure und optional Schwefelsäure in den Reaktor (I);
   (ii) Fahren mindestens eines Anlagenteiles so, dass der Ausgangsstrom des jeweiligen Anlagenteils als Eingangsstrom des jeweiligen Anlagenteils oder eines davor liegenden Anlagenteils verwendet wird (Kreislauffahrweise);
   (iii) Außerbetriebnahme mindestens eines Anlagenteils.

Insbesondere bevorzugt handelt es sich bei der Anlage um die erfindungsgemäße Anlage zur Herstellung von Nitrobenzol.

In einer bevorzugten Ausführungsform umfasst dieses erfindungsgemäße Verfahren die weiteren Schritte:
(iv) Optional Öffnen des in Schritt (iii) außer Betrieb genommenen mindestens einen Anlagenteils;
(v) Durchführung einer Instandsetzungs-, Reinigungs-, Revisions- und/oder Reparaturmaßnahme;
(vi) Optional Schließen und optional Inertisieren des mindestens einen Anlagenteils aus Schritt (v);
(vii) Inbetriebnahme des mindestens einen Anlagenteils aus Schritt (vi);
(viii) Starten der Zufuhr von Benzol und Salpetersäure in den Reaktor (I).

Besonders bevorzugt wird jedes Anlagenteil, welches nicht abgefahren (außer Betrieb genommen) wurde, auf Kreislauffahrweise umgestellt und somit vorteilhaft die Vorteile und Effekte der vorliegenden Erfindung erreicht.

In einer erfindungsgemäßen Anlage kann das erfindungsgemäße Verfahren beispielsweise folgendermaßen durchgeführt werden:
Im ersten Schritt werden zeitgleich der Benzol- und Salpetersäureeingangsstrom abgestellt. Die Kreislaufschwefelsäure kann dann unter Reaktionsstartbedingungen (bevorzugt 101 °C) mittels der Schwefelsäurekreislaufpumpen durch die Nitratoren, Phasentrennung, Blitzverdampfer (Schwefelsäureaufkonzentrierung) in die Schwefelsäurevorlage und von dort im Kreis zurück zu den Nitratoren gestellt werden.

Im zweiten Schritt werden die Wäschen, nachdem das gewaschene Rohnitrobenzol aus der neutralen Wäsche zum Rohnitrotank, über die saure Wäsche und alkalische Wäsche zurück zur neutralen Wäsche geführt wird, in Kreislauffahrweise gestellt.

Im dritten Schritt wird die Destillation zusammen mit den Wäschen in Kreislauffahrweise gestellt.

Im vierten Schritt wird die saure Abwasseraufarbeitung in Kreislauffahrweise gestellt.

Im fünften Schritt wird die alkalische Abwasseraufarbeitung mit Abwasserkolonne und TDZ in Kreislauffahrweise gestellt.

Das von beispielsweise einer Instandhaltungsmaßnahme betroffene Anlagenteil wird abgefahren, entleert, gereinigt und gegebenenfalls für die anstehende Maßnahme geöffnet. Dann wird die Instandhaltungsmaßnahme durchgeführt und das Anlagenteil wird wieder geschlossen, inertisiert und mit Hilfsstoffen und Einsatzmaterialien befüllt und zum Anfahren vorbereitet.

Um die Anlage wieder anzufahren, wird zuerst die saure Abwasseraufarbeitung die Wäschen mit Destillation und anschließend die Nitrierung in Betrieb genommen. Die alkalische Abwasseraufarbeitung wird je nach Bedarf in Betrieb genommen, da sie durch einen Puffertank (Abwasservorlagetank) von der restlichen Anlage abgekoppelt ist.

Das Wiederanfahren der Anlage aus der Kreislauffahrweise heraus kann beispielsweise wie folgt durchgeführt werden:
1.) In der sauren Abwasseraufarbeitung, bestehend aus einem Abwasservorlagetank, einem Wärmetauscher, einer Abwasserdestillation mit Kondensationssystem, einem Abwasserkühler und Ablauf zur sauren Wäsche, fällt nach der Abstellung der Nitrierung wieder saures Abwasser an. Die Abwasserdestillation wird in Betrieb genommen, indem das saure Abwasser im Sumpf der Abwasserkolonne zum Abwasseranal gestellt wird.
2.) In den Wäschen, bestehend aus einem Rohnitrobenzoltank, einer sauren, alkalischen und neutralen Wäsche, wird das Rohnitrobenzol aus dem Rohnitrobenzoltank in die saure Wäsche, über die alkalische Wäsche zur neutralen Wäsche gefahren. Das die neutrale Wäsche verlassende Rohnitrobenzol wird auf die Destillation gestellt. Das Waschwasser wird im Gegenstrom in die neutrale über die alkalische Wäsche zur alkalischen Abwasseraufarbeitung gefahren. Dabei wird der Mengenstrom des Rohnitrobenzols aus dem Rohnitrobenzoltank zur sauren Wäsche und des Waschwassers zur neutralen Wäsche auf die Anfahrlast hochgezogen.
3.) In der Destillation, bestehend aus einem Wärmetauscher, Nitrobenzol-Kolonne mit Sumpfentnahme des Endproduktes Nitrobenzol und einem Naturumlaufverdampfer, Brüdenkondensatoren mit Vakuumpumpen, Brüdenphasentrennapparat, Rückbenzolvorlage mit Pumpe, fällt wieder Rohnitrobenzol an. Der Sumpfablauf der Nitrobenzol-Kolonne wird über den Wärmetauscher auf den Endprodukttank gestellt. Die anfallenden Brüden (Benzol und Wasser) werden kondensiert und im Brüdenphasentrennapparat in eine wässrige und organische Phase getrennt. Die Benzol enthaltende organische Phase wird zum Rückbenzoltank gefördert. Die wässrige Phase wird weiterhin über die saure Wäsche und die saure Abwasseraufarbeitung entsorgt.
4.) In der Nitrierreaktion mit Phasentrennung und Aufkonzentrierung der Schwefelsäure, bestehend aus Nitratoren mit Schwefelsäurekreislaufpumpen, Phasentrennapparat, Blitzverdampfer mit Vakuumpumpen und Kondensatoren und Schwefelsäurevorlage, wird die Kreislaufschwefelsäure weiterhin unter Reaktionsstartbedingungen (bevorzugt 101 °C) mittels der Schwefelsäurekreislaufpumpen durch die Nitratoren, Phasentrennung, Blitzverdampfer (Schwefelsäureaufkonzentrierung) zurück in die Schwefelsäurevorlage gefahren. Zeitgleich werden der Benzolstrom und der Salpetersäurestrom angestellt. Das im Phasentrennapparat anfallende Rohnitrobenzol wird dem Rohnitrobenzoltank zugeführt.
5.) In der alkalischen Abwasseraufarbeitung, bestehend aus einem Abwasservorlagetank, einem Wärmetauscher, einer Abwasserdestillation mit Kondensationssystem, und Ablauf zu einer Anlage zur thermischen Druckzersetzung (TDZ), fällt nach dem Anfahren der alkalischen Wäsche wieder alkalisches Abwasser an. Das alkalische Abwasser im Sumpf der Abwasserkolonne wird auf die TDZ gestellt und die anfallenden Brüden werden weiterhin zur sauren Wäsche gefahren. Die TDZ, bestehend aus Abwasservorlage mit Hochdruckpumpe, Wärmetauscher, Dampfheizer, Verweilzeitrohr und Kühler, wird nun mit gestripptem alkalischen Abwasser beschickt und der Ausgang der TDZ wird auf den Abwasserkanal gestellt.

Die komplette Nitrobenzol-Anlage läuft jetzt mit reduzierter Last (Anfahrlast) und kann nun auf die gewünschte Sollproduktion hochgefahren werden.

Es ist hierbei besonders bevorzugt die Produktionsanlage mit reduzierter Last anzufahren, da ansonsten die benötigten Temperaturprofile für die Nitrierung, Wäschen, Abwasseraufarbeitung und Destillation nicht schnell genug zur Verfügung stehen, was zu unvollständiger Nitrierung mit erhöhten Nebenprodukten und erschwerten Aufarbeitungsbedingungen führen könnte.

Die Umstellung auf Kreislauffahrweise kann beispielsweise für die Nitrierreaktion mit Phasentrennung und Aufkonzentrierung der Schwefelsäure, Wäschen (Rohnitrobenzoltank, saure, alkalische und neutrale Wäsche), Destillation und saure Abwasseraufarbeitung und alkalische Abwasseraufarbeitung wie folgt durchgeführt werden:
Die Gesamtanlage wird in einem ersten Schritt (Schritt a)) abgefahren, indem die Benzol- und Salpetersäurezufuhr zum Nitrierreaktor gestoppt wird. Kreislaufschwefelsäure läuft weiter und das noch im Nitrierreaktor entstehende Rohnitrobenzol wird in den Phasentrennapparat gespült. Dort erfolgt die Phasentrennung und Ausschleusung von restlichem Rohnitrobenzol zum Rohnitrobenzoltank. Im Blitzverdampfer wird die Kreislaufschwefelsäure von restlichen Organika befreit und mittels Dampf auf Starttemperatur (ca. 101 °C) gehalten.

Im folgenden **Schritt b)** erfolgt die Umstellung der Wäschen auf Kreislauffahrweise, wobei der Rohnitrobenzol- und der Waschwasserstrom auf Minimallast gestellt werden.

Im sich anschließenden **Schritt c)** erfolgt die Umstellung der Destillation auf Kreislauffahrweise unter Beibehaltung des Vakuums.

In **Schritt d)** erfolgt nach Abstellung der Reaktion die Umstellung der sauren Abwasseraufarbeitung auf Kreislauffahrweise.

In **Schritt e)** erfolgt nach Abstellung der Wäschen aus Schritt b) die Umstellung der alkalischen Abwasseraufarbeitung (inkl. TDZ) auf Kreislauffahrweise.

In **Schritt f)** erfolgt die kurzzeitige Außerbetriebnahme des vom Kurzstillstand betroffenen Anlagenteils und die Durchführung der Kurzstillstandmaßnahme.

In **Schritt g)** wird das vom Kurzstillstand betroffene Anlagenteil wieder betriebsbereit gemacht.

Anschließend wird in **Schritt h)** die Gesamtanlage aus der Kreislauffahrweise heraus wieder angefahren und anschließend auf die angestrebte Solllast gebracht.

Durch das erfindungsgemäße Verfahren ergeben sich die folgenden Vorteile:
i) Erhöhung der Produktivität, weil sich die Verfügbarkeit der Anlage erhöht, da sich der Zeitbedarf für das Abfahren und Wiederanfahren der Anlage für den Produktionsstillstand stark minimiert.
ii) Investitionskosten in eine größere Anlagenkapazität und damit in Zusammenhang stehende Instandhaltungskosten entfallen.
iii) Es kann mit einem kleineren Endprodukttank gearbeitet werden, da weniger Puffervolumen notwendig ist. Dadurch fallen auch geringere Investitionskosten und Instandhaltungskosten für den Endprodukttank an.
iv) In vielen Fällen kommt es zur Einsparung von Energie, weil die zum Wiederanfahren benötigten Vorbereitungen für die ausgeschalteten Anlagenteile wie das Aufwärmen der Einsatz- und Hilfsstoffe oder das Aufwärmen des Equipments etc. entfallen.
v) Einsparung von Hilfsstoffen wie Dampf-Kondensat und Stickstoff.
vi) Die Reparaturanfälligkeit der Pumpen, speziell im Nitrierbereich, und der Vakuumpumpen wird verbessert, da, wenn die Pumpen bei einem Stillstand abgestellt werden, bei jedem Wiederanfahren der Pumpen die Gleitringdichtungen leiden. Somit werden Folgereparaturen vermieden, was sich wiederum positiv auf die Produktivität der Anlage und die Instandhaltungskosten auswirkt.
vii) Vermeidung von überflüssigen Abfallprodukten wie beispielsweise überschüssiges Benzol, überschüssige Salpetersäure bzw. Salpetersäure/Schwefelsäure-Gemische, nitrierte Oxidationsprodukte des Benzols und Mischungen mit Rohprodukten, Abwasser, die zusätzlich aufgereinigt werden müssen, die entstehen, wenn die Anlage komplett neu angefahren werden muss.

Der Erfolg der erfindungsgemäßen Vorgehensweise ist für den Fachmann überraschend, weil er, um grundsätzlich Energie einzusparen und um sich auf die anstehenden Instandhaltungsmaßnahmen im Produktionsstillstand konzentrieren zu können, viel eher dazu tendieren würde, *die Gesamtanlage* abzustellen, zumal für das erfindungsgemäße Verfahren bzw. für die erfindungsgemäße Anlage Zusatzinvestitionen in rückführende Rohrleitungen samt Pumpen, Umbauten an den Apparaten sowie zusätzliche Prozessleittechnik in Kauf genommen werden.

Im Folgenden soll die vorliegende Erfindung durch Beispiele veranschaulicht werden.

### Beispiele

Gehaltsangaben in ppm oder % sind Massenanteile bezogen auf die Gesamtmasse des jeweiligen Stoff(strom)s. Analysenwerte wurden, sofern nicht anders angegeben, mittels Gaschromatographie bestimmt.

### Allgemeine Bedingungen für die Herstellung von Nitrobenzol bei einer "eingefahrenen" Produktionsanlage

In einen Nitrierreaktor wurden bei einer Produktionslast von 50 t/h Nitrobenzol ein Schwefelsäure-, ein Salpetersäure-, ein Frischbenzol- und ein Rückbenzolstrom eindosiert. Es wurde ein 5 bis 10 %iger Überschuss an Benzol, bezogen auf Salpetersäure, eingesetzt. Nach vollständiger Umsetzung der Salpetersäure mit dem Benzol zu Nitrobenzol unter adiabater Reaktionsführung wurde das nunmehr ca. 130 °C heiße Reaktionsprodukt einem Phasentrennapparat zugeführt, in dem das Reaktionsprodukt in eine organische Phase (= Roh-Nitrobenzol, enthaltend neben Nitrobenzol noch Benzol) und eine wässrige Phase (= Absäure, enthaltend neben Schwefelsäure noch geringe Anteile an Nitrobenzol und Benzol) zerfiel. Die wässrige, hauptsächlich Schwefelsäure umfassende Phase wurde im Verdampfer durch plötzliche Druckerniedrigung einer Blitzverdampfung von Wasser unterzogen und auf diese Weise aufkonzentriert. Die aufkonzentrierte Schwefelsäure wurde im Schwefelsäuretank zur erneuten Verwendung gelagert. Nach dem Abtrennen im Phasentrennapparat wurde das Rohnitrobenzol in der Rohnitrobenzolkühlung auf ca. 50 °C abgekühlt und der Wäsche zugeführt. Diese Wäsche umfasste eine saure, eine alkalische und eine neutrale Waschstufe.

Der so erhaltene von Nitrophenolen und Salzen weitgehend befreite Strom gereinigten RohNitrobenzols wurde wieder aufgeheizt und in einer Destillationskolonne von Wasser, Benzol und anderen Leichtsiedern, welche über Kopf abgetrennt wurden, befreit, wodurch getrocknetes Rein-Nitrobenzol erhalten wurde. Das kondensierte Kopfprodukt der Destillationskolonne wurde einem Phasentrennapparat zugeführt, in dem das Kopfprodukt in eine organische Phase (enthaltend Benzol) und eine wässrige Phase zerfiel. Die organische Phase wurde in einem Puffertank zwischengelagert und von dort, wie oben schon beschrieben, zur Reaktion in den Zulauf des Nitrierreaktors zurückgefahren. Der Stromverbrauch einer derartigen Anlage liegt bei ca. 890 kW/h.

Das in der alkalischen Wäsche anfallende Abwasser wurde wie folgt aufgearbeitet:
Das Abwasser aus der alkalischen Wäsche wurde in einen Absetztank gefahren, in dem ungelöstes Benzol und Nitrobenzol abgeschieden wurden. 3,5 Tonnen pro Stunde an alkalischem Abwasser, das einen Gehalt an Nitrobenzol von 2870 ppm, an Benzol von 409 ppm und an Nitrophenolen von 11809 ppm sowie einen pH-Wert von 12,8 hatte (1,8 % NaOH-Überschuss gegenüber dem Ausgangsgehalt an Nitrophenolen vor der alkalischen Wäsche) wurden in eine Strippkolonne gefahren, um aus diesem alkalischen Abwasser Benzol und Nitrobenzol durch Strippen mit Wasserdampf über Kopf zu entfernen. Dafür wurden 500 kg/h 6-bar-Dampf verwendet. Der Druck im Kolonnenkopf betrug 1,05 bar (absolut), und die Temperatur lag bei 99,5 °C. Der Kopf der Strippkolonne war mit einem vertikalen Kondensator bestückt, in dem die Benzol und Nitrobenzol enthaltenden Brüden auskondensiert anschließend in die saure Wäsche zurückgeführt wurden. Das feuchte, 99 °C heiße Abgas der Strippkolonne war direkt auf dem Kondensator angebunden und mit 30 °C warmen, sauren Wasser aus dem Sauerwassertank abgespritzt. Dadurch wurde die mögliche Ablagerung von Ammoniumnitrat und/oder Ammoniumnitrit, die bei der Verwendung einer konventionellen Abgasleitung für die separate Ableitung des Abgases aus dem Kondensator im trockenen Bereich einer solchen Abgasleitung entstehen können, verhindert (die genannten Ammoniumsalze können aus in dem alkalischen Abwasser befindlichen Ammoniak und Stickoxiden entstehen). Das saure Wasser wurde zusammen mit den auskondensierten Brüden der sauren Wäsche zugeführt. Eine Fehlfunktion der Strippkolonne kann beispielsweise durch redundante Sicherheitseinrichtungen überwacht werden. Nach der Strippung wurde ein alkalisches Abwasser erhalten, das Benzol nur noch in einer Konzentration von bis zu 10 ppm und Nitrobenzol in einer Konzentration von bis zu 10 ppm enthielt. Anschließend wurde das so behandelte alkalische Abwasser in einer Anlage zur thermischen Druckzersetzung bei einer Verweilzeit von 20 min, einer Temperatur von 290 °C und einem absoluten Druck von 90 bar behandelt. Das dabei entstandene Abwasser wurde auf 80 °C abgekühlt. Danach wurde das Abwasser mit Direktdampf gestrippt. Im Sumpf der Strippkolonne wurde ein Strom von 4,0 Tonnen pro Stunde bei einem absoluten Druck von 1,02 bar erhalten, der im Wesentlichen Wasser, Ammoniak, Kohlendioxid und Organika enthielt. Das Kopfprodukt wurde kondensiert und auf 35 °C abgekühlt. Aus dem Kondensat wurde ein Purge-Strom an Organika ausgeschleust. 0,25 Tonnen pro Stunde des an Organika abgereicherten wässrigen Kondensat-Stromes wurden als Rückfluss in die Strippkolonne zurückgeführt. Der Anteil an Organika im erhaltenen Abwasser, das einer biologischen Kläranlage zugeführt wurde, betrug 4726 ppm. Der Gehalt an Ammonium im Abwasser war kleiner als 87 ppm. Es gab keinerlei Probleme mit Ablagerungen im Bereich des Abgases der Strippkolonne.

Auf diese Weise hergestelltes Nitrobenzol hat typischerweise eine Reinheit von ca. 99,96 % (GC), einen Restbenzolgehalt von 0,0028 % (GC), einen Gehalt an 1,3-Dinitrobenzol von 0,0273 % (GC) und einen Nitrophenolgehalt von < 5 ppm (HPLC). Ferner weist das Nitrobenzol einen Wassergehalt von 0,0079 % (bestimmt nach Karl-Fischer) auf.

**Beispiel 1 (Vergleichsbeispiel):** Kurzstillstand einer Produktionsanlage mit Komplettabstellung der Anlage, Reinigungsmaßnahme und Wiederanstellung der Anlage.

Der Kurzstillstand der Anlage diente dazu, Reinigungsarbeiten im Nitrierbereich durchzuführen. Hierfür wurde die Anlage komplett abgefahren, also Nitrierung, Wäschen, Destillation, Sauerwasseraufarbeitung und alkalische Abwasseraufarbeitung. Die Energiezufuhren während den Reinigungsarbeiten wurden abgestellt. Nach den Reinigungsarbeiten wurde wieder angefahren, wobei die komplette Anlage vorab inertisiert, befüllt und aufgeheizt werden musste.

### Durchführung der Komplettabstellung der Anlage:

Als erstes wurde die Nitrierung abgestellt: Die Dosierpumpen der Eingangsströme von Benzol und Salpetersäure wurden abgestellt. Der Dampf des Blitzverdampfers wurde 5 Minuten nach den Rohstoffen Benzol und Salpetersäure abgestellt. Die Kreislaufschwefelsäure lief 1 Stunde weiter, bis alle Organika aus dem Nitrierkreislauf, bestehend aus Nitratoren, Phasentrennapparat, Blitzverdampfer und Kreislaufschwefelsäurevorlagetank, ausgetragen worden waren. Dann wurde der 100 °C heiße Schwefelsäurekreislauf durch Abstellen der Kreislaufpumpe unterbrochen. Die Nitratoren, der Phasentrennapparat und der Blitzverdampfer blieben unter Schwefelsäure stehen. Die restliche Kreislaufschwefelsäure befand sich im Schwefelsäurevorlagetank. Das Gesamtinventar an Schwefelsäure betrug 74 Tonnen. Gleichzeitig mit der Kreislaufpumpe wurde die Vakuumpumpe zum Blitzverdampfer abgestellt und das Vakuum mit Stickstoff aufgehoben. Nun stand der Nitrierkreislauf. Zeitbedarf 2 Stunden, ohne Vakuum aufheben 1 Stunde.

Danach wurde die Sauerwasseraufarbeitung abgestellt, indem die Einspeisung des sauren Abwassers aus dem Sauerwasservorlagetank zum Sauerwasserstripper unterbrochen wurde. Der Dampf zum Sauerwasserstripper und die Sumpfpumpe des Strippers wurden abgestellt. Nun stand die Sauerwasseraufarbeitung. Der Zeitbedarf war 5 Minuten.

Als nächstes wurden die Wäschen abgestellt, indem die Rohnitrobenzolzufuhr aus dem Rohnitrobenzoltank zur sauren Wäsche unterbrochen wurde. Der Rohnitrobenzolweg durch die saure, alkalische und neutrale Wäsche wurde abgestellt, indem die Förderpumpen des Rohnitrobenzols vor den jeweiligen Wäschen gestoppt wurden. Die Wäschen wiesen eine Betriebstemperatur von 48 °C auf und blieben mit Rohnitrobenzol gefüllt. Gleichzeitig wurden der saure, der alkalische und der neutrale Waschwasserweg durch Abschalten der jeweiligen Pumpen abgestellt. Der Zeitbedarf war 5 Minuten.

Dann wurde die Destillation abgestellt, indem die Einspeisung von Rohnitrobenzol unterbrochen wurde und der Dampf zur Destillationskolonne weggenommen wurde. Unmittelbar danach wurde der Produktaustrag durch Abstellen der Sumpfpumpe unterbrochen und der Rücklauf am Kolonnenkopf durch Stoppen der Benzolpumpe abgestellt. Nachdem die Vakuumpumpe abgestellt und mit Stickstoff belüftet war, stand die Destillation. Der Zeitbedarf war 5 Minuten.

Als letztes wurde die alkalische Abwasseraufarbeitung abgestellt, indem die thermische Druckzersetzung auf Kreislauffahrweise gestellt wurde und der Dampf zur Druckzersetzung abgestellt wurde. Gleichzeitig wurden die Zufuhr und der Ablauf des alkalischen Abwassers des Strippers durch Ausschalten der Abwasserpumpen gestoppt und der Dampf zum Stripper geschlossen. Der Zeitbedarf war 5 Minuten. Die Kreislauffahrweise der thermischen Druckzersetzung (TDZ) wurde nach 10 Stunden gestoppt, sobald das Kreislaufwasser unter 100 °C abgekühlt war.

Die Komplettabstellung dauerte ohne Spülung und Entleerung der Apparate, Pumpen und Rohrleitungen insgesamt zwei Stunden, wenn man vom Abfahren (Kaltfahren) der TDZ absieht. In modernen, automatisierten Produktionsanlagen spielt die Personalstärke für den Abfahrprozess nur eine untergeordnete Rolle.

### Durchführung der Reinigungsmaßnahme:

Es war eine Verstopfung im Benzolvorwärmer im Ablauf des Rohnitrobenzols vom Phasentrennapparat zum Rohnitrotank zu beseitigen:
Zuerst wurde der Stand im Phasentrennbehälter um 50 % abgesenkt, damit keine Organik in den zu reinigenden Benzolvorwärmer nachlaufen konnte. Anschließend wurde der Benzolvorwärmer über einen Spülstutzen, der zwischen Phasentrennapparat und Benzolvorwärmer angebracht wurde, für 1 Stunde mit Kondensat gespült, um Rohnitrobenzol und Spuren an Schwefelsäure zu entfernen. Das Spülkondensat wurde zur sauren Wäsche abgeleitet. Danach wurde der Benzolvorwärmer mechanisch von Zu- und Ablauf getrennt und schwarze Niederschläge im Benzolvorwärmer, die die Verstopfung ausmachten, wurden mit großen Mengen Kondensat mit zwei weiteren Spülstutzen zur Kläranlage herausgespült. Der Zeitbedarf lag bei 3 Stunden. Nach Demontage aller 3 Spülstutzen wurden der Zu- und Ablauf des Benzolvorwärmers montiert. Der Zeitbedarf hierfür war 2 Stunden. Danach wurden die betroffenen Rohrleitungen mehrfach aufgewärmt und abgekühlt und dabei die Flanschverbindungen mit den neuen Dichtungen nachgezogen. Der Zeitbedarf betrug 2 Stunden.

Die Reinigungsmaßnahme dauerte insgesamt 8 Stunden. In modernen, automatisierten Produktionsanlagen spielt die Personalstärke für die Vorbereitung der Reinigungsmaßnahme, nämlich Teilentleerung der Anlage, Montage des Spülstutzens zur Vorreinigung des Benzolvorwärmers mit Zu- und Ablauf und anschließender Vorreinigung mit Kondensat, eine wichtige Rolle. In diesem Fall wird ein zusätzlicher Produktionsmitarbeiter benötigt. Handwerker zur Demontage und Montage der Rohrleitungen für die Reinigungsmaßnahme und das Reinigungspersonal selbst werden ebenfalls benötigt.

### Durchführung der Wiederanstellung der Anlage:

Vorab wurden in der gesamten Produktionsanlage die Vakuumpumpen in Betrieb genommen. Der Phasentrennapparat und der gereinigte Benzolvorwärmer wurden mit 100 Nm³ Stickstoff inertisiert.

Als erstes wurden die Wäschen angestellt, indem durch Anstellen der Rohnitrobenzolpumpe die Rohnitrobenzolzufuhr aus dem Rohnitrobenzoltank zur sauren Wäsche in Betrieb genommen wurde. Danach wurden der saure, der alkalische und der neutrale Waschwasserweg durch Einschalten der jeweiligen Pumpen angestellt. Dann wurde der Rohnitrobenzolweg durch die saure, alkalische und neutrale Wäsche angestellt, indem die Förderpumpen des Rohnitrobenzols vor den jeweiligen Wäschen eingeschaltet wurden. Die Waschapparate, die mit Rohnitrobenzol und Waschwasser gefüllt waren, hatten 45 °C und wärmten sich nach dem Anstellen der Produktionsanlage langsam wieder auf 48 °C auf.
Nachdem die letzte Stufe der neutralen Wäsche durch Einspeisen von 3 t/h Kondensat in Betrieb genommen war, wurde die Destillation angestellt, indem Vakuum auf die Destillationskolonne gegeben und aus der letzten neutralen Wäsche 45 °C warmes Rohnitrobenzol zur Destillationskolonne gefahren wurden. Danach wurde die Sumpfpumpe der Kolonne angestellt und Rohnitrobenzol zum Rohnitrobenzoltank gefahren. Nun wurde die Destillationskolonne mit 2 t/h 16 bar Dampf beaufschlagt und auf 170 °C aufgeheizt. Bei 50 °C im Kopf der Kolonne wurde der Rücklauf durch Anstellen der Benzolpumpe in Betrieb genommen. Die Wäschen und die Destillation waren nach 4,5 Stunden zum Wiederanfahren der Produktionsanlage bereit.

Parallel zu den Wäschen und der Destillation wurde die Sauerwasseraufarbeitung angestellt, indem 1 t/h 6 bar Dampf zum Aufwärmen des Sauerwasserstrippers aufgegeben wurden und die Sumpfpumpe des Strippers angestellt wurde. Anschließend wurde die Einspeisung des sauren Abwassers aus dem Sauerwasservorlagetank zum Sauerwasserstripper angestellt. Nun stand die Sauerwasseraufarbeitung zum Wiederanfahren der Produktionsanlage bereit. Der Zeitbedarf zum Anstellen des Sauerwasserstrippers inklusive der Analytik des sauren Abwassers auf Organika mittels Gaschromatographie war 1 Stunde.

Parallel zu den Wäschen und der Destillation wurde die alkalische Abwasseraufarbeitung angestellt, indem die in Kreislauffahrweise gestellte thermische Druckzersetzung mit 0,6 t/h 110 bar Dampf beaufschlagt wurde, um das Kreislaufwasser von 85 °C auf 285 °C zu bringen. 2 Stunden vor dem Ausschleusen des alkalischen Abwassers wurde der Stripper mit 0,5 t/h 6 bar Dampf beaufschlagt und die Zufuhr und der Ablauf des alkalischen Abwassers des Strippers wurden durch Anschalten der Abwasserpumpen angestellt. Der Zeitbedarf war 8 Stunden.

Eineinhalb Stunden, bevor die Wäschen und die Destillation zum Wiederanfahren der Produktionsanlage bereit waren und nachdem die Sauerwasseraufarbeitung in Kreislauffahrweise lief, wurde die Schwefelsäurekreislaufpupe angestellt und die Schwefelsäure über den Nitrator, Phasentrennapparat, Blitzverdampfer und Schwefelsäurevorlagetank im Kreis gefahren. Im Blitzverdampfer wurde das Vakuum angestellt und anschließend 2,4 t/h 6 bar Dampf aufgegeben, womit die Kreislaufschwefelsäure auf Starttemperatur erhitzt wurde. Dieser Vorgang dauerte 1 Stunde, bis die auf 93 °C abgekühlte Kreislaufschwefelsäure auf 100 °C aufgewärmt war.

Nach 4,5 Stunden waren die Wäschen und die Destillation betriebsbereit und die Produktionsanlage wurde durch Anstellen der Benzol- und Salpetersäurepumpen mit 50 % der Nennkapazität gestartet, was einer Produktionsleistung von 25 t/h Nitrobenzol entsprach. Nach 1 Minute kam im Phasentrennapparat das Reaktionsprodukt an und der Sauerwasserstripper wurde auf Ausschleusen des sauren Abwassers gestellt und die Sumpfkolonne der Destillation wurde auf Produktaustrag des Endproduktes Nitrobenzol gestellt. Das Hochziehen der Produktionsanlage auf Nennlast, das in einer modernen Produktionsanlage automatisiert ist, dauert nochmals 1 Stunde.

### Bilanz des Energie-, Hilfsstoff- und Zeitbedarfes für das Ab- und Anfahren der Anlage

### inklusive der Reinigungsmaßnahme:

Der gesamte Zeitbedarf für die Maßnahme betrug 15 Stunden. Dies gilt für den Fall, wenn genügend Personal vorhanden ist und keine technischen Schwierigkeiten auftreten. Der Zeitbedarf für die Reinigung an sich betrug 8 Stunden. Für das Abfahren wurden 2,5 Stunden benötigt. Das Anfahren dauerte 4,5 Stunden.

Insgesamt gingen so 775 Tonnen an Produktion von Nitrobenzol verloren. Der Dampfverbrauch betrug 4,4 Tonnen 6 bar Dampf, 8 Tonnen 16 bar Dampf und 4,8 Tonnen 110 bar Dampf. Beim Abfahren der Anlage und während der Maßnahme wurde kein Dampf verbraucht. Der Verbrauch an Stickstoff betrug für das Abfahren 550 Nm³ und für das Wiederanfahren der Anlage 100 Nm³. Der Verbrauch an Kondensat betrug 15,5 m³ (2 m³ für das Spülen des Wärmetauschers und 13,5 m³ für das Anfahren der neutralen Wäsche). Der Verbrauch an Strom betrug insgesamt 6130 kW. Für das Abfahren der Anlage wurden 1100 kW für die TDZ, 180 kW für die Nitrierung und 445 kW für die Wäschen benötigt. Für das Anfahren der Anlage wurden 4005 kW für die Wäschen und 400 kW für die Kreislaufschwefelsäurepumpen benötigt. Während der Reinigungsmaßnahme wurde kein Strom verbraucht.

**Beispiel 2 (erfindungsgemäß):** Kurzstillstand der Produktionsanlage mit Kreislauffahrweise der nicht von der Reinigungsmaßnahme betroffenen Anlagenteile, Reinigungsmaßnahme und Wiederanstellung der Anlage.

Der Kurzstillstand der Anlage diente für Reinigungsarbeiten im Nitrierbereich. Dazu wurde der Nitrierbereich komplett abgefahren und die anderen Anlagenteile wie die Wäschen, die Destillation, die saure und die alkalische Abwasseraufarbeitung in Kreislauffahrweise gestellt. Die Energien während der Reinigungsarbeiten waren nur im Nitrierbereich abgestellt (Das Vakuum verblieb in stand-by). Nach den Reinigungsarbeiten wurde wieder angefahren, wobei nur der Nitrierbereich komplett inertisiert, befüllt und aufgeheizt werden musste.

### Durchführung der Komplettabstellung der Nitrierung und Einstellung der restlichen Anlagenteile auf Kreislauffahrweise:

Als erstes wurde die Sauerwasseraufarbeitung in Kreislauffahrweise gestellt, indem die Ausschleusung des sauren Abwassers in den Abwasserkanal mittels der Sumpfpumpe des Strippers, die 10 kW/h benötigt, zurück in den Sauerwasservorlagetank gestellt wurde. Der Dampf zum Sauerwasserstripper wurde von 1,2 t/h auf 0,7 t/h 6 bar Dampf gedrosselt und die Einspeisung von Sauerwasser aus dem Sauerwasservorlagetank mittels der Sauerwasserpumpe, die 10 kW/h benötigt, in den Sauerwasserstripper wurde von 20 m³ auf 13 m³ reduziert. Das Umstellen der Sauerwasseraufarbeitung auf Kreislauffahrweise erfolgte mittels Automatik in 33 Sekunden.

Als nächstes wurde die alkalische Abwasseraufarbeitung in Kreislauffahrweise gestellt, indem die Ausschleusung des alkalischen Abwassers der thermische Druckzersetzung (TDZ) in den Abwasserkanal mittels der Hochdruckpumpe der TDZ, die 55 kW/h benötigt, zurück in die alkalische Abwasservorlage gestellt wurde. Der Dampf zur Druckzersetzung wurde von 0,32 t/h auf 0,20 t/h 110 bar Dampf gedrosselt und die Einspeisung von alkalischem Abwasser aus der alkalischen Abwasservorlage in die TDZ wurde von 4,0 m³/h auf 2,5 m³/h reduziert. Das Umstellen der TDZ auf Kreislauffahrweise erfolgte mittels Automatik in 5 Minuten, weil die Reduzierung der Einspeisung zur TDZ manuell erfolgte.

Gleichzeitig wurde der Stripper des alkalischen Abwassers in Kreislauffahrweise gestellt, indem die Ausschleusung des alkalischen Abwassers in die TDZ unterbrochen wurde und mittels der Sumpfpumpe des Strippers, die 10 kW/h benötigt, das alkalische Abwasser zurück auf den Abwassertank gestellt wurde. Der Dampf zum Stripper des alkalischen Abwassers wurde von 0,4 t/h auf 0,25 t/h 6 bar Dampf gedrosselt und die Einspeisung von alkalischem Abwasser aus dem Abwassertank in den Stripper des alkalischen Abwassers wurde mittels der Einspritzpumpe des alkalischen Abwassers, die 10 kW/h benötigt, von 4 m³ auf 2,5 m³ reduziert. Das Umstellen des Strippers des alkalischen Abwassers auf Kreislauffahrweise erfolgte mittels Automatik in 27 Sekunden.

Als nächstes wurden die Wäschen und die Destillation in Kreislauffahrweise gestellt, indem die Ausschleusung des Endproduktes Nitrobenzol aus der Nitrobenzol-Kolonne zum Nitrobenzollagertank mittels der Sumpfpumpe der Kolonne, die 24 kW/h benötigt, auf den Rohnitrobenzoltank umgestellt wurde. Gleichzeitig wurde die Benzol enthaltende organische Phase des Brüdenphasentrennapparates über natürlichen Ablauf zum Rohnitrobenzoltank geführt. Die wässrige Phase des Brüdenphasentrennapparates wurde über die saure Wäsche und die saure Abwasseraufarbeitung entsorgt. Das Vakuumsystem der Nitrobenzol-Kolonne blieb in Betrieb. Die Kreislauffahrweise wurde hergestellt, indem der Inhalt des Rohnitrobenzoltanks mittels Förderpumpen über die gesamten Wäschen zurück zur Nitrobenzol-Kolonne gefahren wurde. Der Rohnitrotank, die saure Wäsche, die alkalische Wäsche und die 3 neutralen Wäschen haben jeweils eine Förderpumpe, die jeweils 24 kW/h benötigen. Die Einspeisung von Rohnitrobenzol in die Wäschen bzw. die Destillation wurde von 42 t/h auf 27 t/h reduziert. Der Dampf zur Nitrobenzol-Kolonne wurde von 2,5 t/h auf 1,6 t/h 16 bar Dampf gedrosselt. Das Waschwasser zur neutralen Wäsche wurde von 6,3 m³/h auf 4,0 m³/h reduziert. Das Umstellen der Wäschen und der Destillation auf Kreislauffahrweise erfolgte mittels Automatik in 5 Minuten,
Als letztes wurde die Nitrierung abgestellt, indem die Dosierpumpen der Eingangsströme von Benzol und Salpetersäure abgestellt wurden. Der Dampf des Blitzverdampfers wurde 5 Minuten nach den Rohstoffen Benzol und Salpetersäure abgestellt. Die Kreislaufschwefelsäure lief 1 Stunde weiter, bis alle Organika aus dem Nitrierkreislauf, bestehend aus Nitratoren, Phasentrennapparat, Blitzverdampfer und Kreislaufschwefelsäurevorlagetank, ausgetragen waren. Dann wurde der 100 °C heiße Schwefelsäwekreislauf durch Abstellen der Kreislaufpumpe unterbrochen. Die Nitratoren, der Phasentrennapparat und der Blitzverdampfer blieben unter Schwefelsäure stehen. Die restliche Kreislaufschwefelsäure befand sich im Schwefelsäurevorlagetank. Gleichzeitig mit der Kreislaufpumpe wurde die Vakuumpumpe zum Blitzverdampfer abgestellt und das Vakuum mit 350 Nm³ Stickstoff aufgehoben. Nun stand der Nitrierkreislauf. Der Zeitbedarf für die Abstellung betrug 2 Stunden.

Die Vorbereitung (Einstellen Kreislauffahrweise von Wäsche, Destillation, alkalisches und saures Abwasser und das Abstellen der Nitrierung) für die Reinigungsmaßnahme dauerte ohne Spülung und Entleerung der Apparate, Pumpen und Rohrleitungen insgesamt 2 Stunden und 11 Minuten.

**Durchführung der Reinigungsmaßnahme:** die Reinigungsmaßnahme wurde, wie in Beispiel 1 beschrieben, durchgeführt.

### Durchführung der Wiederanstellung der Anlage:

Vorab wurde die Vakuumpumpe für den Blitzverdampfer in Betrieb genommen. Der Phasentrennapparat und der gereinigte Benzolvorwärmer wurden mit 100 Nm³ Stickstoff inertisiert. Die Inbetriebnahme der Anlage wurden mit dem Anfahren der Nitrierung gestartet, indem die Schwefelsäurekreislaufpumpe angestellt und die Schwefelsäure über den Nitrator, Phasentrennapparat, Blitzverdampfer und Schwefelsäurevorlagetank im Kreis gefahren wurde. Im Blitzverdampfer war das Vakuum bereits angestellt und es wurden 2,4 t/h 6 bar Dampf aufgegeben, womit die Kreislaufschwefelsäure auf Starttemperatur erhitzt wurde. Dieser Vorgang dauerte 1 Stunde, bis die auf 93 °C abgekühlte Kreislaufschwefelsäure auf 100 °C aufgewärmt war. Nun wurde die Nitrierung durch Anstellen der Benzol- und Salpetersäurepumpen mit 50 % der Nennkapazität gestartet, was einer Produktionsleistung von 25 t/h Nitrobenzol entsprach. Nach 1 Minute kam im Phasentrennapparat das Reaktionsprodukt an, der Sauerwasserstripper wurde auf Ausschleusen des sauren Abwassers gestellt und die Sumpfkolonne der Destillation wurde auf Produktaustrag des Endproduktes Nitrobenzol gestellt. Gleichzeitig wurde der Stripper der alkalischen Abwasseraufarbeitung auf Ausschleusung zur TDZ und die TDZ von Kreislauffahrweise auf Ausschleusung in den Abwasserkanal gestellt. Das Hochziehen der Produktionsanlage auf Nennlast, das in einer modernen Produktionsanlage automatisiert ist, dauerte nochmals 1 Stunde.

### Bilanz des Energie- und Zeitbedarfes für Ab- und Anfahren aus Kreislauffahrweise der Anlage inklusive der Reinigungsmaßnahme:

Der gesamte Zeitbedarf für die Maßnahme betrug 11 Stunden und 12 Minuten. Dieses gilt insbesondere für den Fall, wenn genügend Personal vorhanden ist und keine technischen Schwierigkeiten auftreten. Der Zeitbedarf für die Reinigung an sich betrug 8 Stunden. Für das Abfahren in Kreislauffahrweise wurden 2 Stunden und 11 Minuten benötigt. Das Anfahren aus der Kreislauffahrweise dauerte 1 Stunde und 1 Minute.

Insgesamt gingen auf diese Weise 585 Tonnen an Produktion von Nitrobenzol verloren. Der Dampfverbrauch betrug 12 Tonnen 6 bar Dampf, 13 Tonnen 16 bar Dampf und 1,6 Tonnen 110 bar Dampf für die Kreislauffahrweise. Beim Abfahren der Anlage in die Kreislauffahrweise wurde kein Dampf verbraucht. Der Verbrauch an Stickstoff betrug für das Abfahren in Kreislauffahrweise 350 Nm³ und für das Wiederanfahren der Anlage aus Kreislauffahrweise 100 Nm³. Der Verbrauch an Kondensat für die Maßnahme betrug 47 m³ (2 m³ für das Spülen des Wärmetauschers und 45 m³ für An- und Abfahren und Kreislauffahrweise der Wäschen). Der Verbrauch an Strom betrug insgesamt 8525 kW. Für das Abfahren der Anlage wurden 1943 kW, für die Kreislauffahrweise während der Reinigungsmaßnahme 5680 kW für die Kreislauffahrweise und für das Anfahren der Anlage 905 kW an Strom verbraucht.

### Fazit der Komplettabstellung (Vergleichsbeispiel 1) versus Kreislauffahrweise (Beispiel 2) für die Reinigungsmaßnahme:

Als Fazit der Komplettabstellung (Vergleichsbeispiel 1) versus Kreislauffahrweise (Beispiel 2) kann festgestellt werden, dass der Mehrbedarf an Strom und Kondensat durch den Minderverbrauch an Stickstoff, aber vor allen Dingen durch die höhere Verfügbarkeit der Anlage, die sich durch eine höhere Produktionsleistung bemerkbar macht, mehr als kompensiert wird. Der Dampfverbrauch hält sich in etwa die Waage. Die für die Reinigungsmaßnahme gesparte Zeit beträgt 3 Stunden und 48 Minuten, was einer verbesserten Produktionsleistung von 190 Tonnen Nitrobenzol entspricht.

**Beispiel 3 (Vergleichsbeispiel):** Kurzstillstand der Produktionsanlage mit Komplettabstellung der Anlage, Reparaturmaßnahme und Wiederanstellung der Anlage.

Kurzstillstand der Anlage für eine Reparaturmaßnahme in der Wäsche: Dazu wurde die Anlage komplett abgefahren, also Nitrierung, Wäschen und Destillation. Die Energien waren während der Reparaturmaßnahme abgestellt. Nach der Reparatur wurde wieder angefahren, wobei die komplette Anlage inertisiert, befüllt und aufgeheizt werden musste.

### Durchführung der Komplettabstellung der Anlage:

Die Anlage wurde, wie in Beispiel 1 beschrieben, abgestellt. Die Komplettabstellung dauerte ohne Spülung und Entleerung der Apparate, Pumpen und Rohrleitungen wieder zwei Stunden, wenn man vom Abfahren (Kaltfahren) der TDZ absieht.

### Durchführung der Reparaturmaßnahme:

Undichte Rohrleitung in der neutralen Wäsche abdichten: Dafür wurde die betroffene Rohrleitung in den nachgeschalteten Apparat der Wäsche mit 10 m³ Stickstoff leergeblasen. Dann wurde mit 2 m³ Kondensat gespült und die Rohrleitung entleert. Anschließend wurde die defekte Dichtung der Rohrleitung ersetzt. Die Reparaturmaßnahme dauerte insgesamt 1,5 Stunden. In modernen, automatisierten Produktionsanlagen spielt die Personalstärke für die Vorbereitung der Reparatur, nämlich das Spülen der Rohrleitung, eine wichtige Rolle. In diesem Fall wird ein zusätzlicher Produktionsmitarbeiter benötigt. Handwerker zur Demontage und Montage der Rohrleitung, um die defekte Dichtung zu ersetzten, werden ebenfalls benötigt.

### Durchführung der Wiederanstellung der Anlage:

Vorab wurden in der gesamten Produktionsanlage die Vakuumpumpen in Betrieb genommen. Anschließend wurde die Anlage, wie in Beispiel 1 beschrieben, wieder angestellt. Die Anlage war nach 4,5 Stunden wieder angefahren und konnte auf Nennlast hochgezogen werden.

### Bilanz des Energie- und Zeitbedarfes für Ab- und Anfahren der Anlage inklusive der Reinigungsmaßnahme:

Der gesamte Zeitbedarf für die Maßnahme betrug 8,5 Stunden, da genügend Personal vorhanden war und keine technischen Schwierigkeiten auftraten. Der Zeitbedarf für die Reparatur an sich betrug 1,5 Stunden. Für das Abfahren wurden 2,5 Stunden benötigt. Das Anfahren dauerte 4,5 Stunden. Insgesamt gingen so 450 Tonnen an Produktion von Nitrobenzol verloren. Der Dampfverbrauch betrug 3,4 Tonnen 6 bar Dampf und 8 Tonnen 16 bar Dampf und 4,8 Tonnen 110 bar Dampf. Beim Abfahren der Anlage wurde kein Dampf verbraucht. Es wurden insgesamt 610 Nm³ Stickstoff benötigt, davon 550 Nm³ für das Abfahren und 50 Nm³ für das Wiederanfahren der Anlage, weitere 10 Nm³ für die Reparaturmaßnahme.

Der Verbrauch an Kondensat betrug 15,5 m³ (2 m³ für das Spülen der Rohrleitung und 13,5 m³ für das Anfahren der neutralen Wäsche). Der Verbrauch an Strom betrug insgesamt 5470 kW. Für das Abfahren der Anlage wurden 440 kW für die TDZ, 180 kW für die Nitrierung und 445 kW für die Wäschen benötigt. Für das Anfahren der Anlage wurden 4005 kW für die Wäschen und 400 kW für die Kreislaufschwefelsäurepumpen benötigt. Während der Reparatunnaßnahme wurde kein Strom verbraucht.

**Beispiel 4 (erfindungsgemäß):** Kurzstillstand der Produktionsanlage mit Kreislauffahrweise der nicht von der Reparaturmaßnahme betroffenen Anlagenteile, Reparaturmaßnahme und Wiederanstellung der Anlage.

Kurzstillstand der Anlage für Reparaturarbeiten in der Nitrobenzolwäsche: Dazu wurden die Wäschen und die Destillation komplett abgefahren. Die anderen Anlagenteile wie die Nitrierung, die saure und die alkalische Abwasseraufarbeitung wurden in Kreislauffahrweise gestellt. Die Energien während der Reparaturarbeiten waren nur in der Wäsche und der Destillation abgestellt (Vakuum blieb in stand-by). Nach der Reparatur wurde wieder angefahren.

### Durchführung der Komplettabstellung der Wäschen und Destillation und Einstellung der

### restlichen Anlagenteile auf Kreislauffahrweise:

Als erstes wurde die Sauerwasseraufarbeitung in Kreislauffahrweise gestellt, indem die Ausschleusung des sauren Abwassers in den Abwasserkanal mittels der Sumpfpumpe des Strippers, die 10 KW/h benötigt, zurück in den Sauerwasservorlagetank gestellt wurde. Der Dampf zum Sauerwasserstripper wurde von 1,2 t/h auf 0,7 t/h 6 bar Dampf gedrosselt und die Einspeisung von Sauerwasser aus dem Sauerwasservorlagetank mittels der Sauerwasserpumpe, die 10 KW/h benötigt, in den Sauerwasserstripper wurde von 20 m³ auf 13 m³ reduziert. Das Umstellen der Sauerwasseraufarbeitung auf Kreislauffahrweise erfolgte mittels Automatik in 29 Sekunden.

Als nächstes wurde die alkalische Abwasseraufarbeitung in Kreislauffahrweise gestellt, indem die Ausschleusung des alkalischen Abwassers der thermische Druckzersetzung (TDZ) in den Abwasserkanal mittels der Hochdruckpumpe der TDZ, die 55 KW/h benötigt, zurück in die alkalische Abwasservorlage gestellt wurde. Der Dampf zur Druckzersetzung wurde von 0,32 t/h auf 0,20 t/h 110 bar Dampf gedrosselt und die Einspeisung von alkalischem Abwasser aus der alkalischen Abwasservorlage in die TDZ wurde von 4,0 m³/h auf 2,5 m³/h reduziert. Das Umstellen der TDZ auf Kreislauffahrweise erfolgte mittels Automatik in 5 Minuten, weil die Reduzierung der Einspeisung zur TDZ manuell erfolgte.

Gleichzeitig wurde der Stripper des alkalischen Abwassers in Kreislauffahrweise gestellt, indem die Ausschleusung des alkalischen Abwassers in die TDZ unterbrochen wurde und mittels der Sumpfpumpe des Strippers, die 10 kW/h benötigt, das alkalische Abwasser zurück auf den Abwassertank gestellt wurde. Der Dampf zum Stripper des alkalischen Abwassers wurde von 0,4 t/h auf 0,25 t/h 6 bar Dampf gedrosselt und die Einspeisung von alkalischem Abwasser aus dem Abwassertank in den Stripper des alkalischen Abwassers wurde mittels der Einspritzpumpe des alkalischen Abwassers, die 10 kW/h benötigt, von 4 m³ auf 2,5 m³ reduziert. Das Umstellen des Strippers des alkalischen Abwassers auf Kreislauffahrweise erfolgte mittels Automatik in 31 Sekunden.

Als nächstes wurde die Einsatzstoffe zur Nitrierung abgestellt. Die Dosierpumpen der Eingangsströme von Benzol und Salpetersäure wurden abgestellt. Die Kreislaufschwefelsäure lief bei 100 °C weiter über die Nitratoren, den Phasentrennapparat, Blitzverdampfer und Kreislaufschwefelsäurevorlagetank im Kreis. Am Blitzverdampfer wurden 0,3 t/h 6 bar Dampf benötigt. Der Zeitbedarf war 1 Minute.

Als letztes wurden die Wäschen abgestellt, indem die Rohnitrobenzolzufuhr aus dem Rohnitrobenzoltank zur sauren Wäsche unterbrochen wurde. Der Rohnitrobenzolweg durch die saure, alkalische und neutrale Wäsche wurde abgestellt, indem die Förderpumpen des Rohnitrobenzols vor den jeweiligen Wäschen gestoppt wurden. Die Wäschen hatten 48 °C und blieben mit Rohnitrobenzol gefüllt. Gleichzeitig wurden der saure, der alkalische und der neutrale Waschwasserweg durch Abschalten der jeweiligen Pumpen abgestellt. Der Zeitbedarf lag bei 5 Minuten.

Dann wurde die Destillation abgestellt, indem die Einspeisung von Rohnitrobenzol unterbrochen und der Dampf zur Destillationskolonne weggenommen wurde. Unmittelbar danach wurde der Produktaustrag durch Abstellen der Sumpfpumpe unterbrochen und der Rücklauf am Kolonnenkopf durch Stoppen der Benzolpumpe abgestellt. Die Vakuumpumpe lief weiter. Der Zeitbedarf war 5 Minuten.

Die Vorbereitung (Einstellung der Kreislauffahrweise der Nitrierung und der alkalischen und sauren Abwasseraufarbeitung und das Abstellen der Wäschen und der Destillation) für die Reparaturmaßnahme dauerte ohne Spülung und Entleerung der Apparate, Pumpen und Rohrleitungen insgesamt 11 Minuten.

### Durchführung der Reparaturmaßnahme:

Die Reparaturmaßnahme wurde wie in Beispiel 3 beschrieben, durchgeführt. Der Zeitbedarf lag wiederum bei 1,5 Stunden. Der Dampfverbrauch während der Kreislauffahrweise betrug 1,6 Tonnen 6 bar Dampf und 0,4 Tonnen 110 bar Dampf. 400 kW Strom wurden für den Betrieb der Vakuumpumpe und Schwefelsäurekreislaufpumpe während der Kreislauffahrweise benötigt.

### Durchführung der Wiederanstellung der Anlage:

Als erstes wurden die Wäschen angestellt, indem durch Anstellen der Rohnitrobenzolpumpe die Rohnitrobenzolzufuhr aus dem Rohnitrobenzoltank zur sauren Wäsche in Betrieb genommen wurde. Danach wurden der saure, der alkalische und der neutrale Waschwasserweg durch Einschalten der jeweiligen Pumpen angestellt. Dann wurde der Rohnitrobenzolweg durch die saure, alkalische und neutrale Wäsche angestellt, indem die Förderpumpen des Rohnitrobenzols vor den jeweiligen Wäschen eingeschaltet wurden. Die Waschapparate, die mit Rohnitrobenzol und Waschwasser gefüllt waren, hatten 45 °C und erwärmten sich nach dem Anstellen der Produktionsanlage langsam wieder auf 48 °C.

Nachdem die letzte Stufe der neutralen Wäsche durch Einspeisen von 3 t/h Kondensat in Betrieb genommen war, wurde die Destillation angestellt, indem aus der letzten neutralen Wäsche 45 °C warmes Rohnitrobenzol zur Destillationskolonne gefahren wurde. Danach wurde die Sumpfpumpe der Kolonne angestellt und Rohnitrobenzol zum Rohnitrobenzoltank gefahren. Nun wurde die Destillationskolonne mit 2 t/h 16 bar Dampf beaufschlagt und auf 170 °C aufgeheizt. Bei 50 °C im Kopf der Kolonne wurde der Rücklauf durch Anstellen der Benzolpumpe in Betrieb genommen. Die Wäschen und die Destillation waren nach 1 Stunde zum Wiederanfahren der Produktionsanlage bereit.

Nun wurde die Nitrierung durch Anstellen der Benzol- und Salpetersäurepumpen mit 50 % der Nennkapazität gestartet, was einer Produktionsleistung von 25 t/h Nitrobenzol entsprach. Nach 1 Minute kam im Phasentrennapparat das Reaktionsprodukt an, der Sauerwasserstripper wurde auf Ausschleusen des sauren Abwassers und die Sumpfkolonne der Destillation auf Produktaustrag des Endproduktes Nitrobenzol gestellt. Gleichzeitig wurde der Stripper der alkalischen Abwasseraufarbeitung auf Ausschleusung zur TDZ und die TDZ von Kreislauffahrweise auf Ausschleusung in den Abwasserkanal gestellt. Das Hochziehen der Produktionsanlage auf Nennlast dauerte nochmals 1 Stunde.

### Bilanz des Energie- und Zeitbedarfes für Ab- und Anfahren aus Kreislauffahrweise der

### Anlage inklusive der Reparaturmaßnahme:

Der gesamte Zeitbedarf für die Maßnahme betrug 3 Stunden und 41 Minuten. Der Zeitbedarf für die Reparatur an sich betrug 1,5 Stunden. Für das Abfahren in Kreislauffahrweise wurden 11 Minuten benötigt. Das Anfahren aus der Kreislauffahrweise dauerte 1 Stunde und 1 Minute.

Insgesamt gingen so 165 Tonnen an Produktion von Nitrobenzol verloren. Der Dampfverbrauch betrugl,6 Tonnen 6 bar Dampf, 2 Tonnen 16 bar Dampf und 0,8 Tonnen 110 bar Dampf. Beim Abfahren der Anlage in Kreislauffahrweise wurde kein Dampf verbraucht. Es wurden 10 Nm³ Stickstoff für die Reparaturmaßnahme und weitere 50 Nm³ Stickstoff für das Wiederanfahren der Anlage benötigt. Der Verbrauch an Kondensat betrug 7 m³ (2 m³ für das Spülen der Rohrleitung und 5 m³ für das Ab- und Anfahren der neutralen Wäsche. Der Verbrauch an Strom betrug insgesamt 1593 KW. Für das Abfahren der Anlage wurden 178 kW für das Herunterfahren der Nitrierung in Kreislauffahrweise, 510 kW für die Kreislauffahrweise während der Reinigungsmaßnahme und 905 kW an Strom für das Anfahren der Anlage verbraucht.

### Fazit der Komplettabstellung (Vergleichsbeispiel 3) versus Kreislauffahrweise (Beispiel 4) für

### die Reparaturmaßnahme:

Als Fazit der Komplettabstellung (Vergleichsbeispiel 3) versus Kreislauffahrweise (Beispiel 4) kann festgestellt werden, dass bei der Kreislauffahrweise geringere Mengen an Dampf, Strom, Stickstoff und Kondensat verbraucht wurden und obendrein die Verfügbarkeit der Anlage, die sich durch eine höhere Produktionsleistung bemerkbar macht, deutlich besser war. Die für die Reparaturmaßnahme gesparte Zeit betrug 5 Stunden und 48 Minuten, was einer verbesserten Produktionsleistung von 290 Tonnen Nitrobenzol entspricht.

## Patentansprüche

1. Bevorzugt adiabatisch betriebenes Verfahren zur Herstellung von Nitrobenzol, umfassend die Schritte
(I) Nitrierung von Benzol mit Salpetersäure in Schwefelsäure unter Bildung von Nitrobenzol in einem Reaktor, wobei Benzol mit einem Massenstrom m₁, Salpetersäure mit einem Massenstrom m₂ und Schwefelsäure mit einem Massenstrom m₃ in den Reaktor eingetragen werden;
(II) Phasentrennung des Reaktionsgemisches aus Schritt (I) in einem Phasentrennapparat in eine wässrige, Schwefelsäure enthaltende Phase und eine organische, Nitrobenzol enthaltende Phase; sowie optional (und bevorzugt) die Schritte (III) bis (VII):
(III) Aufkonzentration der in Schritt (II) erhaltenen wässrigen Phase durch Verdampfung von Wasser in einem Verdampfungsapparat zu einer wässrigen Schwefelsäure-haltigen Phase mit erhöhter Schwefelsäurekonzentration, und wobei die aufkonzentrierte, Schwefelsäure-haltige, wässrige Phase über einen Schwefelsäuretank in Schritt (I) zurückgeführt und als Bestandteil des Massenstroms m₃ eingesetzt wird;
(IV) Mindestens zweistufiges Waschen der in Schritt (II) erhaltenen organischen, Nitrobenzol enthaltenden Phase und Abtrennung der wässrigen Phase nach jeder Stufe, wobei in jeder Stufe ein Waschbehälter mit Phasentrenneinrichtung oder ein Waschbehälter und ein separater Phasentrennapparat eingesetzt werden,
(V) Destillation, bevorzugt Rektifikation der in der letzten Stufe von Schritt (IV) erhaltenen organischen, Nitrobenzol enthaltenden Phase in einem Destillationsapparat, und
(VI) Aufarbeitung des Abwassers der ersten Waschstufe von Schritt (IV) umfassend Sammeln des Abwassers in einem Abwassersammelbehälter und Reinigung dieses Abwassers in einer Vorrichtung zur Destillation oder Strippung,
(VII) Aufarbeitung des Abwassers der zweiten Waschstufe von Schritt (IV) umfassend Sammeln des Abwassers in einem Abwassersammelbehälter und Reinigung dieses Abwassers in einer Vorrichtung zur Destillation oder Strippung, wobei der Vorrichtung zur Destillation oder Strippung eine Vorrichtung zur thermischen Druckzersetzung vor- und/oder nachgeschaltet sein kann,
**dadurch gekennzeichnet, dass**
bei einer Außerbetriebnahme eines oder mehrerer Anlagenteile aus den Schritten (I) bis (VII), insofern diese durchgeführt werden, der Massenstrom m₁ und der Massenstrom m₂ auf null reduziert werden und in mindestens einem der nicht außer Betrieb genommenen Anlagenteile der Ausgangsstrom wieder als Eingangsstrom des jeweiligen Anlagenteils oder eines davor liegenden Anlagenteils verwendet wird.

2. Verfahren gemäß Anspruch 1, umfassend die Schritte (III) bis (VII).

3. Verfahren gemäß Anspruch 2, wobei Schritt (IV) die Schritte
(IVa) Waschen der in Schritt (II) erhaltenen organischen, Nitrobenzol enthaltenden Phase in mindestens einer, bevorzugt einer bis zwei, besonders bevorzugt einer Wäsche(n) und anschließende Phasentrennung in eine wässrige und eine organische, Nitrobenzol enthaltende Phase,
(IVb) Waschen der in Schritt (IVa) erhaltenen organischen, Nitrobenzol enthaltenden Phase in mindestens einer, bevorzugt einer bis zwei, besonders bevorzugt einer alkalischen Wäsche(n) mit einer wässrigen Lösung einer Base ausgewählt aus der Gruppe bestehend aus Natriumhydroxid, Natriumcarbonat und Natriumhydrogencarbonat, und anschließende Phasentrennung in eine wässrige und eine organische, Nitrobenzol enthaltende Phase,
(IVc) Waschen der in Schritt (IVb) erhaltenen organischen, Nitrobenzol enthaltenden Phase in mindestens einer, bevorzugt zwei bis vier, besonders bevorzugt zwei bis drei, ganz besonders bevorzugt zwei neutralen Wäsche(n) mit Wasser und anschließende Phasentrennung in eine wässrige und eine organische, Nitrobenzol enthaltende Phase,
umfasst.

4. Verfahren nach mindestens einem der vorstehenden Ansprüche, wobei in jedem nicht außer Betrieb genommenen Anlagenteil der Ausgangsstrom wieder als Eingangsstrom des entsprechenden Anlagenteils oder eines davor liegenden Anlagenteils verwendet wird.

5. Verfahren nach mindestens einem der vorstehenden Ansprüche, wobei der Ausgangsstrom des Anlagenteils aus Schritt (V) als Eingangsstrom des Anlagenteils aus Schritt (IV) verwendet wird.

6. Anlage zur Herstellung von Nitrobenzol, umfassend die Anlagenteile:
(I) einen Reaktor zur Nitrierung von Benzol mit einem Gemisch aus Salpetersäure und Schwefelsäure unter Bildung von Nitrobenzol,
(II) einen Phasentrennapparat zur Phasentrennung des im Reaktor (I) erhaltenen Reaktionsgemisches in eine wässrige, Schwefelsäure enthaltende Phase und eine organische, Nitrobenzol enthaltende Phase, sowie optional (und bevorzugt) die Anlagenteile (III) bis (VII):
(III) einen Verdampfer (III.a) zur Aufkonzentration der wässrigen, Schwefelsäure enthaltenden Phase und einen Schwefelsäurevorlagetank (III.b) zur Aufnahme der aufkonzentrierten, wässrigen, Schwefelsäure enthaltenden Phase und deren Bereitstellung für den Reaktor (I),
(IV) einen Waschbehälter mit Phasentrenneinrichtung pro Waschstufe oder einen Waschbehälter und einen separaten Phasentrennapparat pro Waschstufe zum mindestens zweistufigen Waschen der organischen, Nitrobenzol enthaltenden Phase aus (II),
(V) einen Destillationsapparat zur Reinigung der organischen, Nitrobenzol enthaltenden Phase aus (IV),
(VI) einen Abwassersammelbehälter und eine Vorrichtung zur Destillation oder Strippung zum Sammeln und anschließenden Reinigen des Abwassers der ersten Waschstufe aus (IV),
(VII) einen Abwassersammelbehälter und eine Vorrichtung zur Destillation oder Strippung, welcher eine Vorrichtung zur thermischen Druckzersetzung vor- und/oder nachgeschaltet sein kann, zum Sammeln und anschließenden Reinigen des Abwassers der zweiten Waschstufe aus (IV),
wobei
die Anlage derart ausgestaltet ist, dass bei einer Außerbetriebnahme eines oder mehrerer Anlagenteile (I) bis (VII), insofern diese vorhanden sind, kein weiterer Eintrag von Benzol und Salpetersäure in den Reaktor (I) stattfindet und unabhängig voneinander oder gleichzeitig in mindestens einem nicht von der Außerbetriebnahme betroffenen Anlagenteil der Ausgangsstrom zurückgeführt und als Eingangsstrom des jeweiligen Anlagenteils oder eines davor liegenden Anlagenteils verwendet wird.

7. Anlage gemäß Anspruch 6, umfassend die Anlagenteile (III) bis (VII).

8. Anlage gemäß Anspruch 7, wobei das Anlagenteil (IV) umfasst:
(IVa) einen Waschbehälter mit Phasentrenneinrichtung oder einen Waschbehälter und einen separaten Phasentrennapparat zur Wäsche und anschließenden Phasentrennung der organischen, Nitrobenzol enthaltenden Phase aus (II),
(IVb) einen Waschbehälter mit Phasentrenneinrichtung oder einen Waschbehälter und einen separaten Phasentrennapparat zur Wäsche und anschließenden Phasentrennung der organischen, Nitrobenzol enthaltenden Phase aus (IVa),
(IVc) einen Waschbehälter mit Phasentrenneinrichtung oder einen Waschbehälter und einen separaten Phasentrennapparat zur Wäsche und anschließenden Phasentrennung der organischen, Nitrobenzol enthaltenden Phase aus (IVb).

9. Anlage gemäß mindestens einem der Ansprüche 6 bis 8, wobei unabhängig voneinander oder gleichzeitig in jedem nicht außer Betrieb genommenen Anlagenteil Ausgangsstrom zurückgeführt und wieder als Eingangsstrom des jeweiligen Anlagenteiles oder eines davor liegenden Anlagenteils werden kann.

10. Anlage gemäß mindestens einem der Ansprüche 6 bis 9, wobei die Anlage so ausgestaltet ist, dass der Ausgangsstrom des Anlagenteils (V) als Eingangsstrom des Anlagenteils (IV) verwendet werden kann.

11. Verfahren zum Betrieb einer Anlage zur Herstellung von Nitrobenzol, umfassend die Anlagenteile:
(I) einen Reaktor zur Nitrierung von Benzol mit einem Gemisch aus Salpetersäure und Schwefelsäure unter Bildung von Nitrobenzol,
(II) einen Phasentrennapparat zur Phasentrennung des im Reaktor (I) erhaltenen Reaktionsgemisches in eine wässrige, Schwefelsäure enthaltende Phase und eine organische, Nitrobenzol enthaltende Phase, sowie optional (und bevorzugt) die Anlagenteile (III) bis (VII):
(III) einen Verdampfer (III.a) zur Aufkonzentration der wässrigen, Schwefelsäure enthaltenden Phase und einen Schwefelsäurevorlagetank (III.b) zur Aufnahme der aufkonzentrierten, wässrigen, Schwefelsäure enthaltenden Phase und deren Bereitstellung für den Reaktor (I),
(IV) einen Waschbehälter mit Phasentrenneinrichtung pro Waschstufe oder einen Waschbehälter und einen separaten Phasentrennapparat pro Waschstufe zum mindestens zweistufigen Waschen der organischen, Nitrobenzol enthaltenden Phase aus (II),
(V) einen Destillationsapparat zur Reinigung der organischen, Nitrobenzol enthaltenden Phase aus (IV),
(VI) einen Abwassersammelbehälter und eine Vorrichtung zur Destillation oder Strippung zum Sammeln und anschließenden Reinigen des Abwassers der ersten Waschstufe aus (IV),
(VII) einen Abwassersammelbehälter und eine Vorrichtung zur Destillation oder Strippung, welcher eine Vorrichtung zur thermischen Druckzersetzung vor- und/oder nachgeschaltet sein kann, zum Sammeln und anschließenden Reinigen des Abwassers der zweiten Waschstufe aus (IV),
wobei zur Außerbetriebnahme eines oder mehrerer Anlagenteile (I) bis (VII), insofern diese vorhanden sind, die folgenden Schritte durchlaufen werden:
(i) Stoppen der Zufuhr von Benzol und Salpetersäure und optional Schwefelsäure in den Reaktor (I);
(ii) Fahren mindestens eines Anlagenteiles so, dass der Ausgangsstrom des jeweiligen Anlagenteils als Eingangsstrom des jeweiligen Anlagenteils oder eines davor liegenden Anlagenteils verwendet wird;
(iii) Außerbetriebnahme mindestens eines Anlagenteils.

12. Verfahren gemäß Anspruch 11, weiter umfassend die Schritte
(iv) Optional Öffnen des in Schritt (iii) abgefahrenen mindestens einen Anlagenteils;
(v) Durchführung einer Instandsetzungs-, Reinigungs-, Revisions- und/oder Reparaturmaßnahme;
(vi) Optional Schließen und optional Inertisieren des mindestens einen Anlagenteils aus Schritt (v);
(vii) Anfahren des mindestens einen Anlagenteils aus Schritt (vi);
(viii) Starten der Zufuhr von Benzol und Salpetersäure in den Reaktor (I).

13. Verfahren gemäß Anspruch 11 oder Anspruch 12, wobei die Anlage die Anlagenteile (III) bis (V) umfasst und das Anlagenteil (IV) umfasst:
(IVa) einen Waschbehälter mit Phasentrenneinrichtung oder einen Waschbehälter und einen separaten Phasentrennapparat zur Wäsche und anschließenden Phasentrennung der organischen, Nitrobenzol enthaltenden Phase aus (II),
(IVb) einen Waschbehälter mit Phasentrenneinrichtung oder einen Waschbehälter und einen separaten Phasentrennapparat zur Wäsche und anschließenden Phasentrennung der organischen, Nitrobenzol enthaltenden Phase aus (IVa),
(IVc) einen Waschbehälter mit Phasentrenneinrichtung oder einen Waschbehälter und einen separaten Phasentrennapparat zur Wäsche und anschließenden Phasentrennung der organischen, Nitrobenzol enthaltenden Phase aus (IVb).

14. Verfahren nach mindestens einem der Ansprüche 11 bis 13, wobei in jedem nicht außer Betrieb genommenen Anlagenteil der Ausgangsstrom wieder als Eingangsstrom des entsprechenden Anlagenteils oder eines davor liegenden Anlagenteils verwendet wird.

15. Verfahren nach mindestens einem der Ansprüche 11 bis 14, wobei der Ausgangsstrom des Anlagenteils (V) als Eingangsstrom des Anlagenteils (IV) verwendet wird.

## Claims

1. Process, preferably operated adiabatically, for preparing nitrobenzene, comprising the steps of
(I) nitrating benzene with nitric acid in sulfuric acid to form nitrobenzene in a reactor, with introduction of benzene with a mass flow rate of m₁, nitric acid with a mass flow rate of m₂ and sulfuric acid with a mass flow rate of m₃ into the reactor;
(II) separating the phases of the reaction mixture from step (I) in a phase separation apparatus into an aqueous sulfuric acid-containing phase and an organic nitrobenzene-containing phase; and optionally (and preferably) steps (III) to (VII):
(III) concentrating the aqueous phase obtained in step (II) by evaporating water in an evaporation apparatus to give an aqueous sulfuric acid-containing phase having elevated sulfuric acid concentration, with recycling of the concentrated sulfuric acid-containing aqueous phase via a sulfuric acid tank into step (I) and use thereof as a constituent of mass flow m₃;
(IV) washing the organic nitrobenzene-containing phase obtained in step (II) in at least two stages and separating off the aqueous phase after each stage, using a wash vessel having a phase separation unit or a wash vessel and a separate phase separation apparatus in each stage,
(V) distilling, preferably rectifying, the organic nitrobenzene-containing phase obtained in the last stage of step (IV) in a distillation apparatus, and
(VI) working up the wastewater from the first wash stage of step (IV), comprising collecting the wastewater in a wastewater collection vessel and cleaning this wastewater in an apparatus for distillation or stripping,
(VII) working up the wastewater from the second wash stage of step (IV), comprising collecting the wastewater in a wastewater collection vessel and cleaning this wastewater in an apparatus for distillation or stripping, where the apparatus for distillation or stripping may be connected up- and/or downstream of an apparatus for thermal pressure decomposition,
**characterized in that**
in the event of shutdown of one or more plant sections from steps (I) to (VII), if they are conducted, the mass flow m₁ and the mass flow m₂ are reduced to zero and, in at least one of the plant sections that has not been shut down, the output stream is used again as input stream for the respective plant section or an upstream plant section.

2. Process according to Claim 1, comprising steps (III) to (VII).

3. Process according to Claim 2, wherein step (IV) comprises the steps of
(IVa) washing the organic nitrobenzene-containing phase obtained in step (II) in at least one wash, preferably one or two washes, more preferably one wash, then separating the phases into an aqueous phase and an organic nitrobenzene-containing phase,
(IVb) washing the organic nitrobenzene-containing phase obtained in step (IVa) in at least one alkaline wash, preferably one or two alkaline washes, more preferably one alkaline wash, with an aqueous solution of a base selected from the group consisting of sodium hydroxide, sodium carbonate and sodium hydrogencarbonate, then separating the phases into an aqueous phase and an organic nitrobenzene-containing phase,
(IVc) washing the organic nitrobenzene-containing phase obtained in step (IVb) in at least one neutral wash, preferably two to four neutral washes, more preferably two or three neutral washes, most preferably two neutral washes, with water, then separating the phases into an aqueous phase and an organic nitrobenzene-containing phase.

4. Process according to at least one of the preceding claims, wherein the output stream in every plant section that has not been shut down is used again as input stream for the corresponding plant section or an upstream plant section.

5. Process according to at least one of the preceding claims, wherein the output stream from the plant section from step (V) is used as input stream for the plant section from step (IV).

6. Plant for preparation of nitrobenzene, comprising the following plant sections:
(I) a reactor for nitration of benzene with a mixture of nitric acid and sulfuric acid to form nitrobenzene,
(II) a phase separation apparatus for phase separation of the reaction mixture obtained in the reactor (I) into an aqueous sulfuric acid-containing phase and an organic nitrobenzene-containing phase, and optionally (and preferably) plant sections (III) to (VII):
(III) an evaporator (III.a) for concentration of the aqueous sulfuric acid-containing phase and a sulfuric acid reservoir tank (III.b) for accommodation of the concentrated aqueous sulfuric acid-containing phase and the provision thereof for the reactor (I),
(IV) a wash vessel with phase separation unit per wash stage or a wash vessel and a separate phase separation apparatus per wash stage for at least two-stage washing of the organic nitrobenzene-containing phase from (II),
(V) a distillation apparatus for purification of the organic nitrobenzene-containing phase from (IV),
(VI) a wastewater collection vessel and an apparatus for distillation or stripping for collection and subsequent cleaning of the wastewater from the first wash stage from (IV),
(VII) a wastewater collection vessel and an apparatus for distillation or stripping which may be connected up- and/or downstream of an apparatus for thermal pressure decomposition, for collection and subsequent cleaning of the wastewater from the second wash stage from (IV),
wherein
the plant is configured such that, in the event of shutdown of one or more plant sections (I) to (VII), if they are present, no further introduction of benzene and nitric acid into the reactor (I) takes place and, independently of one another or simultaneously, in at least one plant section unaffected by the shutdown, the output stream is recycled and used as input stream for the respective plant section or an upstream plant section.

7. Plant according to Claim 6, comprising plant sections (III) to (VII).

8. Plant according to Claim 7, plant section (IV) comprising:
(IVa) a wash vessel with phase separation unit or a wash vessel and a separate phase separation apparatus for washing and subsequent phase separation of the organic nitrobenzene-containing phase from (II),
(IVb) a wash vessel with phase separation unit or a wash vessel and a separate phase separation apparatus for washing and subsequent phase separation of the organic nitrobenzene-containing phase from (IVa),
(IVc) a wash vessel with phase separation unit or a wash vessel and a separate phase separation apparatus for washing and subsequent phase separation of the organic nitrobenzene-containing phase from (IVb).

9. Plant according to at least one of Claims 6 to 8, wherein, independently of one another or simultaneously, the output stream in every plant section that has not been shut down can be recycled and again as input stream for the respective plant section or an upstream plant section.

10. Plant according to at least one of Claims 6 to 9, wherein the plant is configured such that the output stream from plant section (V) can be used as input stream for plant section (IV).

11. Method of operating a plant for preparation of nitrobenzene, comprising the following plant sections:
(I) a reactor for nitration of benzene with a mixture of nitric acid and sulfuric acid to form nitrobenzene,
(II) a phase separation apparatus for phase separation of the reaction mixture obtained in the reactor (I) into an aqueous sulfuric acid-containing phase and an organic nitrobenzene-containing phase, and optionally (and preferably) plant sections (III) to (VII):
(III) an evaporator (III.a) for concentration of the aqueous sulfuric acid-containing phase and a sulfuric acid reservoir tank (III.b) for accommodation of the concentrated aqueous sulfuric acid-containing phase and the provision thereof for the reactor (I),
(IV) a wash vessel with phase separation unit per wash stage or a wash vessel and a separate phase separation apparatus per wash stage for at least two-stage washing of the organic nitrobenzene-containing phase from (II),
(V) a distillation apparatus for purification of the organic nitrobenzene-containing phase from (IV),
(VI) a wastewater collection vessel and an apparatus for distillation or stripping for collection and subsequent cleaning of the wastewater from the first wash stage from (IV),
(VII) a wastewater collection vessel and an apparatus for distillation or stripping which may be connected up- and/or downstream of an apparatus for thermal pressure decomposition, for collection and subsequent cleaning of the wastewater from the second wash stage from (IV),
wherein shutdown of one or more plant sections (I) to (VII), if they are present, is accomplished by running through the following steps:
(i) stopping the supply of benzene and nitric acid and optionally sulfuric acid into the reactor (I);
(ii) running at least one plant section in such a way that the output stream from the respective plant section is used as input stream for the respective plant section or an upstream plant section;
(iii) shutting down at least one plant section.

12. Method according to Claim 11, further comprising the steps of:
(iv) optionally opening the at least one plant section shut down in step (iii);
(v) conducting a maintenance, cleaning, inspection and/or repair measure;
(vi) optionally closing and optionally inertizing the at least one plant section from step (v);
(vii) starting up the at least one plant section from step (vi);
(viii) starting the supply of benzene and nitric acid into the reactor (I).

13. Method according to Claim 11 or Claim 12, wherein the plant comprises plant sections (III) to (V) and plant section (IV) comprises:
(IVa) a wash vessel with phase separation unit or a wash vessel and a separate phase separation apparatus for washing and subsequent phase separation of the organic nitrobenzene-containing phase from (II),
(IVb) a wash vessel with phase separation unit or a wash vessel and a separate phase separation apparatus for washing and subsequent phase separation of the organic nitrobenzene-containing phase from (IVa),
(IVc) a wash vessel with phase separation unit or a wash vessel and a separate phase separation apparatus for washing and subsequent phase separation of the organic nitrobenzene-containing phase from (IVb).

14. Method according to at least one of Claims 11 to 13, wherein the output stream in every plant section that has not been shut down is used again as input stream for the corresponding plant section or an upstream plant section.

15. Method according to at least one of Claims 11 to 14, wherein the output stream from the plant section (V) is used as input stream for the plant section (IV).

## Revendications

1. Procédé de préférence exploité sous forme adiabatique de fabrication de nitrobenzène, comprenant les étapes suivantes :
(I) la nitration de benzène avec de l'acide nitrique dans de l'acide sulfurique avec formation de nitrobenzène dans un réacteur, le benzène étant introduit dans le réacteur en un courant massique m₁, l'acide nitrique en un courant massique m₂ et l'acide sulfurique en un courant massique m₃ ;
(II) la séparation de phases du mélange réactionnel de l'étape (I) dans un appareil de séparation de phases en une phase aqueuse contenant de l'acide sulfurique et une phase organique contenant du nitrobenzène ; ainsi qu'éventuellement (et de préférence) les étapes (III) à (VII) :
(III) la concentration de la phase aqueuse obtenue à l'étape (II) par évaporation de l'eau dans un appareil d'évaporation en une phase aqueuse contenant de l'acide sulfurique ayant une concentration élevée en acide sulfurique, la phase aqueuse concentrée contenant de l'acide sulfurique étant recyclée dans l'étape (I) par le biais d'une cuve d'acide sulfurique et étant utilisée en tant que constituant du courant massique m₃ ;
(IV) le lavage au moins à deux étapes de la phase organique contenant du nitrobenzène obtenue à l'étape (II) et la séparation de la phase aqueuse après chaque étape, un contenant de lavage comprenant un dispositif de séparation de phases ou un contenant de lavage et un appareil de séparation de phases séparé étant utilisés à chaque étape,
(V) la distillation, de préférence la rectification, de la phase organique contenant du nitrobenzène obtenue lors de la dernière étape de l'étape (IV) dans un appareil de distillation, et
(VI) le traitement de l'eau résiduaire de la première étape de lavage de l'étape (IV), comprenant la collecte de l'eau résiduaire dans un contenant de collecte d'eau résiduaire et la purification de cette eau résiduaire dans un dispositif de distillation ou d'extraction,
(VII) le traitement de l'eau résiduaire de la deuxième étape de lavage de l'étape (IV), comprenant la collecte de l'eau résiduaire dans un contenant de collecte d'eau résiduaire et la purification de cette eau résiduaire dans un dispositif de distillation ou d'extraction, un dispositif pour la décomposition sous pression thermique pouvant être raccordé en amont et/ou en aval du dispositif de distillation ou d'extraction, **caractérisé en ce que**
lors d'une mise hors service d'une ou de plusieurs parties d'unité des étapes (I) à (VII), dans la mesure où celles-ci sont réalisées, le courant massique m₁ et le courant massique m₂ sont réduits à zéro et, dans au moins une des parties d'unité non mises hors service, le courant de sortie est réutilisé en tant que courant d'entrée de la partie d'unité en question ou d'une partie d'unité en amont.

2. Procédé selon la revendication 1, comprenant les étapes (III) à (VII).

3. Procédé selon la revendication 2, dans lequel l'étape (IV) comprend les étapes suivantes :
(IVa) le lavage de la phase organique contenant du nitrobenzène obtenue à l'étape (II) lors d'au moins un, de préférence un à deux, de manière particulièrement préférée un lavage, puis la séparation de phases en une phase aqueuse et une phase organique contenant du nitrobenzène,
(IVb) le lavage de la phase organique contenant du nitrobenzène obtenue à l'étape (IVa) lors d'au moins un, de préférence un à deux, de manière particulièrement préférée un lavage alcalin avec une solution aqueuse d'une base choisie dans le groupe constitué par l'hydroxyde de sodium, le carbonate de sodium et l'hydrogénocarbonate de sodium, puis la séparation de phases en une phase aqueuse et une phase organique contenant du nitrobenzène,
(IVc) le lavage de la phase organique contenant du nitrobenzène obtenue à l'étape (IVb) lors d'au moins un, de préférence deux à quatre, de manière particulièrement préférée deux à trois, de manière tout particulièrement préférée deux lavages neutres avec de l'eau, puis la séparation de phases en une phase aqueuse et une phase organique contenant du nitrobenzène.

4. Procédé selon au moins l'une quelconque des revendications précédentes, dans lequel, dans chaque partie d'unité non mise hors service, le courant de sortie est réutilisé en tant que courant d'entrée de la partie d'unité correspondante ou d'une partie d'unité en amont.

5. Procédé selon au moins l'une quelconque des revendications précédentes, dans lequel, le courant de sortie de la partie d'unité de l'étape (V) est utilisé en tant que courant d'entrée de la partie d'unité de l'étape (IV).

6. Unité pour la fabrication de nitrobenzène, comprenant les parties d'unité suivantes :
(I) un réacteur pour la nitration de benzène avec un mélange d'acide nitrique et d'acide sulfurique avec formation de nitrobenzène,
(II) un appareil de séparation de phases pour la séparation de phases du mélange réactionnel obtenu dans le réacteur (I) en une phase aqueuse contenant de l'acide sulfurique et une phase organique contenant du nitrobenzène, ainsi qu'éventuellement (et de préférence) les parties d'unité (III) à (VII) :
(III) un évaporateur (III.a) pour la concentration de la phase aqueuse contenant de l'acide sulfurique et une cuve de stockage d'acide sulfurique (III.b) pour la réception de la phase aqueuse concentrée contenant de l'acide sulfurique et sa mise à disposition pour le réacteur (I),
(IV) un contenant de lavage comprenant un dispositif de séparation de phases par étape de lavage ou un contenant de lavage et un appareil de séparation de phases séparé par étape de lavage pour le lavage à au moins deux étapes de la phase organique contenant du nitrobenzène de (II),
(V) un appareil de distillation pour la purification de la phase organique contenant du nitrobenzène de (IV),
(VI) un contenant de collecte d'eau résiduaire et un dispositif pour la distillation ou l'extraction pour la collecte, puis la purification de l'eau résiduaire de la première étape de lavage de (IV),
(VII) un contenant de collecte d'eau résiduaire et un dispositif pour la distillation ou l'extraction, en amont et/ou en aval duquel un dispositif pour la décomposition sous pression thermique peut être raccordé, pour la collecte, puis la purification de l'eau résiduaire de la deuxième étape de lavage de (IV),
l'unité étant configurée de sorte que, lors d'une mise hors service d'une ou de plusieurs parties d'unité (I) à (VII), dans la mesure où celles-ci sont présentes, aucun apport supplémentaire de benzène et d'acide nitrique dans le réacteur (I) n'a lieu et le courant de sortie est recyclé dans au moins une partie d'unité non concernée par la mise hors service indépendamment les unes des autres ou simultanément et utilisé en tant que courant d'entrée de la partie d'unité en question ou d'une partie d'unité en amont.

7. Unité selon la revendication 6, comprenant les parties d'unité (III) à (VII).

8. Unité selon la revendication 7, dans laquelle la partie d'unité (IV) comprend :
(IVa) un contenant de lavage comprenant un dispositif de séparation de phases ou un contenant de lavage et un appareil de séparation de phases séparé pour le lavage, puis la séparation de phases de la phase organique contenant du nitrobenzène de (II),
(IVb) un contenant de lavage comprenant un dispositif de séparation de phases ou un contenant de lavage et un appareil de séparation de phases séparé pour le lavage, puis la séparation de phases de la phase organique contenant du nitrobenzène de (IVa),
(IVc) un contenant de lavage comprenant un dispositif de séparation de phases ou un contenant de lavage et un appareil de séparation de phases séparé pour le lavage, puis la séparation de phases de la phase organique contenant du nitrobenzène de (IVb).

9. Unité selon au moins l'une quelconque des revendications 6 à 8, dans laquelle le courant de sortie peut être recyclé dans chaque partie d'unité non mise hors service indépendamment les unes des autres ou simultanément, et réutilisé en tant que courant d'entrée de la partie d'unité en question ou d'une partie d'unité en amont.

10. Unité selon au moins l'une quelconque des revendications 6 à 9, dans laquelle l'unité est configurée de sorte que le courant de sortie de la partie d'unité (V) peut être utilisé en tant que courant d'entrée de la partie d'unité (IV).

11. Procédé d'exploitation d'une unité pour la fabrication de nitrobenzène, comprenant les parties d'unité suivantes :
(I) un réacteur pour la nitration de benzène avec un mélange d'acide nitrique et d'acide sulfurique avec formation de nitrobenzène,
(II) un appareil de séparation de phases pour la séparation de phases du mélange réactionnel obtenu dans le réacteur (I) en une phase aqueuse contenant de l'acide sulfurique et une phase organique contenant du nitrobenzène, ainsi qu'éventuellement (et de préférence) les parties d'unité (III) à (VII) :
(III) un évaporateur (III.a) pour la concentration de la phase aqueuse contenant de l'acide sulfurique et une cuve de stockage d'acide sulfurique (III.b) pour la réception de la phase aqueuse concentrée contenant de l'acide sulfurique et sa mise à disposition pour le réacteur (I),
(IV) un contenant de lavage comprenant un dispositif de séparation de phases par étape de lavage ou un contenant de lavage et un appareil de séparation de phases séparé par étape de lavage pour le lavage à au moins deux étapes de la phase organique contenant du nitrobenzène de (II),
(V) un appareil de distillation pour la purification de la phase organique contenant du nitrobenzène de (IV),
(VI) un contenant de collecte d'eau résiduaire et un dispositif pour la distillation ou l'extraction pour la collecte, puis la purification de l'eau résiduaire de la première étape de lavage de (IV),
(VII) un contenant de collecte d'eau résiduaire et un dispositif pour la distillation ou l'extraction, en amont et/ou en aval duquel un dispositif pour la décomposition sous pression thermique pouvant être raccordé, pour la collecte, puis la purification de l'eau résiduaire de la deuxième étape de lavage de (IV),
les étapes suivantes étant réalisées pour la mise hors service d'une ou de plusieurs parties d'unité (I) à (VII), dans la mesure où celles-ci sont présentes :
(i) l'arrêt de l'introduction de benzène et d'acide nitrique et éventuellement d'acide sulfurique dans le réacteur (I) ;
(ii) l'exploitation d'au moins une partie d'unité de sorte que le courant de sortie de la partie d'unité en question est utilisé en tant que courant d'entrée de la partie d'unité en question ou d'une partie d'unité en amont ;
(iii) la mise hors service d'au moins une partie d'unité.

12. Procédé selon la revendication 11, comprenant en outre les étapes suivantes :
(iv) éventuellement l'ouverture de ladite au moins une partie d'unité arrêtée à l'étape (iii),
(v) la réalisation d'une mesure de remise en état, de nettoyage, de révision et/ou de réparation ;
(vi) éventuellement la fermeture et éventuellement l'inertisation de ladite au moins une partie d'unité de l'étape (v) ;
(vii) le démarrage de ladite au moins une partie d'unité de l'étape (vi) ;
(viii) le début de l'introduction de benzène et d'acide nitrique dans le réacteur (I).

13. Procédé selon la revendication 11 ou la revendication 12, dans lequel l'unité comprend les parties d'unité (III) à (V) et la partie d'unité (IV) :
(IVa) un contenant de lavage comprenant un dispositif de séparation de phases ou un contenant de lavage et un appareil de séparation de phases séparé pour le lavage, puis la séparation de phases de la phase organique contenant du nitrobenzène de (II),
(IVb) un contenant de lavage comprenant un dispositif de séparation de phases ou un contenant de lavage et un appareil de séparation de phases séparé pour le lavage, puis la séparation de phases de la phase organique contenant du nitrobenzène de (IVa),
(IVc) un contenant de lavage comprenant un dispositif de séparation de phases ou un contenant de lavage et un appareil de séparation de phases séparé pour le lavage, puis la séparation de phases de la phase organique contenant du nitrobenzène de (IVb).

14. Procédé selon au moins l'une quelconque des revendications 11 à 13, dans lequel, dans chaque partie d'unité non mise hors service, le courant de sortie est réutilisé en tant que courant d'entrée de la partie d'unité correspondante ou d'une partie d'unité en amont.

15. Procédé selon au moins l'une quelconque des revendications 11 à 14, dans lequel le courant de sortie de la partie d'unité (V) est utilisé en tant que courant d'entrée de la partie d'unité (IV).
